(19) Europäisches Patentamt
European Patent Office
Office européen des brevets

(11) EP 3 018 182 A1

(12) EUROPEAN PATENT APPLICATION
published in accordance with Art. 153(4) EPC

(43) Date of publication:
**11.05.2016 Bulletin 2016/19**

(21) Application number: **14820420.9**

(22) Date of filing: **03.07.2014**

(51) Int Cl.:
*C09K 3/00* (2006.01) *C07D 221/14* (2006.01)
*C08K 5/42* (2006.01) *C08L 101/00* (2006.01)
*G03F 7/004* (2006.01) *G03F 7/038* (2006.01)
*H01L 21/027* (2006.01)

(86) International application number:
**PCT/JP2014/003545**

(87) International publication number:
**WO 2015/001804 (08.01.2015 Gazette 2015/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.07.2013 JP 2013141424**
**18.10.2013 JP 2013216988**

(71) Applicant: **San-Apro Limited**
**Kyoto-shi, Kyoto 605-0995 (JP)**

(72) Inventors:
- **IKEDA, Takuya**
**Kyoto-shi**
**Kyoto 615-8245 (JP)**
- **KIMURA, Hideki**
**Kyoto-shi**
**Kyoto 615-8245 (JP)**
- **SHIBAGAKI, Tomoyuki**
**Kyoto-shi**
**Kyoto 615-8245 (JP)**
- **OKA, Masaaki**
**Kyoto-shi**
**Kyoto 615-8245 (JP)**

(74) Representative: **Kotitschke & Heurung Partnerschaft mbB**
**Eduard-Schmid-Str. 2**
**81541 München (DE)**

# (54) PHOTOACID GENERATOR, AND RESIN COMPOSITION FOR PHOTOLITHOGRAPHY

(57) Provided is a non-ionic photoacid generator having high i-line photosensitivity, excellent heat-resistant stability, and excellent solubility in hydrophobic materials. The present invention is a non-ionic photoacid generator (A) that is characterized by being expressed by general formula (1).
[In formula (1), R1 and R2 each independently represents an alkyl group having 1-18 carbons or a fluoroalkyl group having 1-18 carbons, an alkenyl group having 2-18 carbons, an alkynyl group having 2-18 carbons, an aryl group having 6-18 carbons, a silyl group, or the like; m and n respectively represent the number of R1s and R2s, each number being an integer from 0 to 3, and the total number (m+n) of R1s and R2s being an integer from 1 to 6. The m number of R1s and the n number of R2s may each be the same or different. R3 represents a hydrocarbon having 1-18 carbons (wherein one part or all of the hydrogen may be substituted by fluorine).]

EP 3 018 182 A1

## Description

TECHNICAL FIELD

The present invention relates to a photoacid generator and a resin composition for photolithography. More particularly, it relates to a non-ionic photoacid generator suitable for allowing ultraviolet rays (i-line) to act on the photoacid generator to generate a strong acid, and a resin composition for photolithography containing the same.

BACKGROUND ART

In a field of fine processing typified by semiconductor manufacturing, a photolithography process in which i-line with a wavelength of 365 nm is used as exposure light has hitherto been widely used.

As the resist material used in the photolithography process, for example, a resin composition containing a polymer having a carboxylic acid tert-butyl ester group or a phenolic tert-butyl carbonate group and a photoacid generator has been used. As the photoacid generator, ionic photoacid generators such as a triaryl sulfonium salt (Patent Document 1) and a phenacyl sulfonium salt having a naphthalene skeleton (Patent Document 2) and non-ionic acid generators such as an acid generator having an oximesulfonate structure (Patent Document 3) and an acid generator having a sulfonyldiazomethane structure (Patent Document 4) have been known. By allowing this resist material to be irradiated with ultraviolet rays, the photoacid generator is decomposed to generate a strong acid. Furthermore, by being subjected to post exposure baking (PEB), a tert-butyl ester group or a tert-butyl carbonate group is dissociated from the polymer by virtue of this strong acid, a carboxylic acid or a phenolic hydroxyl group is formed, and a part irradiated with ultraviolet rays becomes readily-soluble in an alkali developing solution. This phenomenon is utilized to perform pattern formation.

However, as the photolithography process becomes finer processing, since the influence of swelling becomes greater when the pattern of a light-unexposed part is swelled due to an alkali developing solution, it is necessary to suppress the resist material from being swelled.

For the purpose of solving these problems, there has been proposed a method of suppressing the swelling of the resist material by allowing an alicyclic skeleton, a fluorine-containing skeleton, or the like to be contained in a polymer in the resist material to make the polymer hydrophobic.

However, with regard to the ionic photoacid generator, since the compatibility against a hydrophobic material containing an alicyclic skeleton, a fluorine-containing skeleton, and the like is insufficient, there is a problem that sufficient resist performance is not exerted since the phase separation occurs in the resist material and the pattern formation results in failure. On the other hand, although the non-ionic photoacid generator is satisfactory in compatibility against a hydrophobic material, there are a problem that the sensitivity to i-line is insufficient and a problem that the allowance range is narrow since the heat-resistant stability is insufficient and the photoacid generator is decomposed by post exposure baking (PEB).

PRIOR ART DOCUMENTS

PATENT DOCUMENTS

Patent Document 1: JP-A-50-151997
Patent Document 2: JP-A-9-118663
Patent Document 3: JP-A-06-67433
Patent Document 4: JP-A-10-213899

SUMMARY OF THE INVENTION

PROBLEMS TO BE SOLVED BY THE INVENTION

Accordingly, an object of the present invention is to provide a non-ionic photoacid generator having high photosensitivity to i-line, being excellent in heat-resistant stability and being excellent in solubility to a hydrophobic material.

SOLUTIONS TO THE PROBLEMS

The present inventors have conducted studies in view of achieving the above-described object, and as a result, the present invention has been completed.

That is, the present invention is directed to a non-ionic photoacid generator (A), being expressed by the following general formula (1); and a resin composition (Q) for photolithography comprising the non-ionic photoacid generator (A).

[Chemical 1]

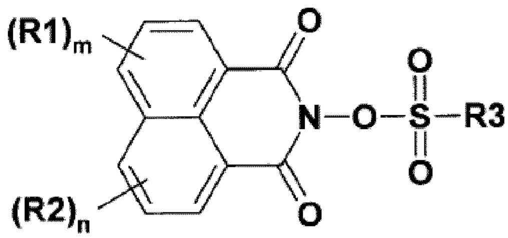

(1)

[In formula (1), R1 and R2 each independently represents an alkyl group having 1 to 18 carbon atoms or a fluoroalkyl group having 1 to 18 carbon atoms, an alkenyl group having 2 to 18 carbon atoms, an alkynyl group having 2 to 18 carbon atoms, an aryl group having 6 to 18 carbon atoms, a silyl group, an alkoxy group or aryloxy group represented by $R^{11}O$-, an alkylthio group or arylthio group represented by $R^{12}S$-, a sulfinyl group represented by $R^{13}SO$-, a sulfonyl group represented by $R^{14}SO_2$-, an alkylcarbonyl group or arylcarbonyl group represented by $R^{15}CO$-, a carbonyloxy group represented by $R^{16}COO$-, an oxycarbonyl group represented by $R^{17}OCO$-, a carbonate group represented by $R^{18}OCOO$-, a urethane group represented by $R^{19}NHCOO$-, or a halogen atom; m and n respectively represent the number of R1s and R2s, each number being an integer from 0 to 3, and the total number (m + n) of R1s and R2s being an integer from 1 to 6. The m number of R1s and the n number of R2s may each be the same or different. R3 represents a hydrocarbon group having 1 to 18 carbon atoms (wherein one part or all of hydrogen atoms may be substituted by fluorine atoms).]

EFFECTS OF THE INVENTION

The non-ionic photoacid generator (A) according to the present invention is non-ionic, thus being excellent in compatibility with a hydrophobic material as compared to an ionic acid generator.

The molar absorptivity to i-line is high because R1 or R2 which is a substituent on the naphthalimide skeleton acts on the electronic state on the naphthalene ring.

Consequently, the non-ionic photoacid generator (A) can be easily decomposed by being irradiated with i-line to generate sulfonic acid which is a strong acid.

Furthermore, the non-ionic photoacid generator (A) has a naphthalimide skeleton, thus being excellent in heat-resistant stability.

As such, the resin composition (Q) for photolithography containing the non-ionic photoacid generator (A) according to the present invention is highly sensitive to i-line, and moreover, is excellent in usability since the allowance range at the time of post exposure baking (PEB) is wide.

MODE FOR CARRYING OUT THE INVENTION

The non-ionic photoacid generator (A) according to the present invention is expressed by the following general formula (1) .

[Chemical 2]

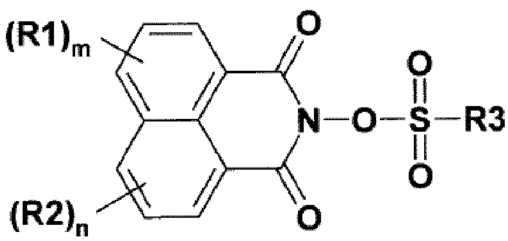

(1)

[In formula (1), R1 and R2 each independently represents an alkyl group having 1 to 18 carbon atoms or a fluoroalkyl group having 1 to 18 carbon atoms, an alkenyl group having 2 to 18 carbon atoms, an alkynyl group having 2 to 18 carbon atoms, an aryl group having 6 to 18 carbon atoms, a silyl group, an alkoxy group or aryloxy group represented by $R^{11}O$-, an alkylthio group or arylthio group represented by $R^{12}S$-, a sulfinyl group represented by $R^{13}SO$-, a sulfonyl group represented by $R^{14}SO_2$-, an alkylcarbonyl group or arylcarbonyl group represented by $R^{15}CO$-, a carbonyloxy group represented by $R^{16}COO$-, an oxycarbonyl group represented by $R^{17}OCO$-, a carbonate group represented by $R^{18}OCOO$-, a urethane group represented by $R^{19}NHCOO$-, or a halogen atom; m and n respectively represent the number of R1s and R2s, each number being an integer from 0 to 3, and the total number (m + n) of R1s and R2s being an integer from 1 to 6. The m number of R1s and the n number of R2s may each be the same or different. R3 represents a hydrocarbon group having 1 to 18 carbon atoms (wherein one part or all of hydrogen atoms may be substituted by fluorine atoms) . ]

The non-ionic photoacid generator (A) according to the present invention is photodecomposed by ultraviolet ray irradiation, particularly by i-line which is exposure light of 365 nm, to generate sulfonic acid which is a strong acid.

R1 and R2, which are essential functional groups for allowing the non-ionic photoacid generator (A) to have an absorption wavelength of i-line which is exposure light of 365 nm and enhancing the solubility to a resist resin, each independently represents an alkyl group having 1 to 18 carbon atoms or a fluoroalkyl group having 1 to 18 carbon atoms, an alkenyl group having 2 to 18 carbon atoms, an alkynyl group having 2 to 18 carbon atoms, an aryl group having 6 to 18 carbon atoms, a silyl group, an alkoxy group or aryloxy group represented by $R^{11}O$-, an alkylthio group or arylthio group represented by $R^{12}S$-, a sulfinyl group represented by $R^{13}SO$-, a sulfonyl group represented by $R^{14}SO_2$-, an alkylcarbonyl group or arylcarbonyl group represented by $R^{15}CO$-, a carbonyloxy group represented by $R^{16}COO$-, an oxycarbonyl group represented by $R^{17}OCO$-, a carbonate group represented by $R^{18}OCOO$-, a urethane group represented by $R^{19}NHCOO$-, or a halogen atom; m and n respectively represent the number of R1s and R2s, each number being an integer from 0 to 3, and the total number (m + n) of R1s and R2s being an integer from 1 to 6. The m number of R1s and the n number of R2s may each be the same or different. R3 is a functional group representing a hydrocarbon group having 1 to 18 carbon atoms (wherein one part or all of hydrogen atoms may be substituted by fluorine atoms), which may have a substituent. R1 and R2 may be the same as or different from each other. The total number (m + n) of R1s and R2s is an integer of 1 to 6.

Examples of the alkyl group having 1 to 18 carbon atoms as said R1 or R2 include a linear alkyl group having 1 to 18 carbon atoms (methyl, ethyl, n-propyl, n-butyl, n-pentyl, n-octyl, n-decyl, n-dodecyl, n-tetradecyl, n-hexadecyl, n-octadecyl or the like), a branched-chain alkyl group having 1 to 18 carbon atoms (isopropyl, isobutyl, sec-butyl, tert-butyl, isopentyl, neopentyl, tert-pentyl, isohexyl and isooctadecyl), and a cycloalkyl group having 3 to 18 carbon atoms (cyclopropyl, cyclobutyl, cyclopentyl, cyclohexyl, 4-decylcyclohexyl or the like).

Examples of the fluoroalkyl group having 1 to 18 carbon atoms as said R1 or R2 include a linear or branched fluoroalkyl group having 1 to 18 carbon atoms (such as a trifluoromethyl, 2,2-difluoroethyl, 2,2,2-trifluoroethyl, pentafluoroethyl, 2,2,3,3-tetrafluoropropyl, 2,2,3,3,3-pentafluoropropyl, 1,1,1,3,3,3-hexafluoro-2-propyl, heptafluoropropyl, 2,2,3,3,4,4,4-heptafluorobutyl, perfluorobutyl, nonafluoro-tert-butyl, 1H,1H-nonafluoropentyl, perfluoropentyl, 1H,1H-tridecafluorohex perfluorohexyl, 1H,1H-pentadecafluorooc and perfluorooctyl group) and the like.

Examples of the alkenyl group having 2 to 18 carbon atoms as said R1 or R2 include a linear or branched one such as vinyl, allyl, 1-propenyl, isopropenyl, 1-butenyl, 2-butenyl, 3-butenyl, 1-methyl-1-propenyl, 1-methyl-2-propenyl, 2-methyl-1-propenyl, 2-methyl-2-propenyl, 1-pentenyl, 2-pentenyl, 3-pentenyl, 4-pentenyl, 1-methyl-1-butenyl, 2-methyl-2-butenyl, 3-methyl-2-butenyl, 1,2-dimethyl-1-propenyl, 1-decenyl, 2-decenyl, 8-decenyl, 1-dodecenyl, 2-dodecenyl and 10-dodecenyl.

Examples of the alkynyl group having 2 to 18 carbon atoms include a linear or branched one such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1,2-dimethyl-2-propynyl, 1-decynyl, 2-decynyl, 8-decynyl, 1-dodecynyl, 2-dodecynyl and 10-dodecynyl.

Examples of the aryl group having 6 to 18 carbon atoms as said R1 or R2 include phenyl, tolyl, dimethylphenyl, naphthyl, anthracenyl, biphenyl, pentafluorophenyl and the like.

Example of the silyl group as said R1 or R2 include a tricarbylsilyl group such as a trimethylsilyl group, an ethyldimethylsilyl group, a methyldiethylsilyl group, a triethylsilyl group, an i-propyldimethylsilyl group, a methyldi-i-propylsilyl group, a tri-i-propylsilyl group, a tert-butyldimethylsilyl group, a methyldi-tert-butylsilyl group, a tri-tert-butylsilyl group, a phenyldimethylsilyl group, a methyldiphenylsilyl group and a triphenylsilyl group.

Examples of the alkoxy group represented by $R^{11}O$- as said R1 or R2 include a linear or branched-chain alkoxy group having 1 to 18 carbon atoms (methoxy, ethoxy, propoxy, isopropoxy, butoxy, isobutoxy, sec-butoxy, tert-butoxy, hexyloxy, decyloxy, dodecyloxy, octadecyloxy or the like) and the like, and an alkoxy group bearing a linear or branched fluoroalkyl group having 1 to 18 carbon atoms (such as a trifluoromethyl, 2,2-difluoroethyl, 2,2,2-trifluoroethyl, pentafluoroethyl, 2,2,3,3-tetrafluoropropyl, 2,2,3,3,3-pentafluoropropyl, 1,1,1,3,3,3-hexafluoro-2-propyl, heptafluoropropyl, 2,2,3,3,4,4,4-heptafluorobutyl, perfluorobutyl, nonafluoro-tert-butyl, 1H,1H-nonafluoropentyl, perfluoropentyl, 1H,1H-tridecafluorohex perfluorohexyl, 1H,1H-pentadecafluorooc and perfluorooctyl group) or the like as R11. Examples of the aryloxy group include phenoxy, naphthoxy, anthroxy, pentafluorophenyloxy, 3-trifluoromethylphenyloxy, 3,5-bistrifluoromethylphenyloxy and the like.

Examples of the alkylthio group represented by $R^{12}S$-as said R1 or R2 include a linear or branched-chain alkylthio group having 1 to 18 carbon atoms (methylthio, ethylthio, propylthio, isopropylthio, butylthio, isobutylthio, sec-butylthio, tert-butylthio, pentylthio, isopentylthio, neopentylthio, tert-pentylthio, octylthio, decylthio, dodecylthio, isooctadecylthio or the like) and the like. Examples of the arylthio group include an arylthio group having 6 to 20 carbon atoms (phenylthio, 2-methylphenylthio, 3-methylphenylthio, 4-methylphenylthio, 2-chlorophenylthio, 3-chlorophenylthio, 4-chlorophenylthio, 2-bromophenylthio, 3-bromophenylthio, 4-bromophenylthio, 2-fluorophenylthio, 3-fluorophenylthio, 4-fluorophenylthio, 2-hydroxyphenylthio, 4-hydroxyphenylthio, 2-methoxyphenylthio, 4-methoxyphenylthio, 1-naphthylthio, 2-naphthylthio, 4-[4-(phenylthio)benzoyl]phenylthio, 4-[4-(phenylthio)phenoxy]phenylthio, 4-[4-(phenylthio)phenyl]phenylthio, 4-(phenylthio)phenylthio, 4-benzoylphenylthio, 4-benzoyl-2-chlorophenylthio, 4-benzoyl-3-chlorophenylthio, 4-benzoyl-3-methylthiophenylthio, 4-benzoyl-2-methylthiophenylthio, 4-(4-methylthiobenzoyl)phenylthio, 4-(2-methylthiobenzoyl)phenylthio, 4-(p-methylbenzoyl)phenylthio, 4-(p-ethylbenzoyl)phenylthio, 4-(p-isopropylbenzoyl)phenylthio, 4-(p-tert-butylbenzoyl)phenylthio or the like) and the like.

Examples of the sulfinyl group represented by $R^{13}SO$-as said R1 or R2 include a linear or branched-chain sulfinyl group having 1 to 18 carbon atoms (methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, tert-pentylsulfinyl, octylsulfinyl, isooctadecylsulfinyl or the like), an arylsulfinyl group having 6 to 10 carbon atoms (phenylsulfinyl, tolylsulfinyl, naphtylsulfinyl or the like) and the like.

Examples of the sulfonyl group represented by $R^{14}SO_2$-as said R1 or R2 include a linear or branched-chain alkylsulfonyl group having 1 to 18 carbon atoms (methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, tert-pentylsulfonyl, octylsulfonyl, octadecylsulfonyl or the like), an arylsulfonyl group having 6 to 10 carbon atoms {phenylsulfonyl, tolylsulfonyl (a tosyl group), naphthylsulfonyl or the like} and the like.

Examples of the alkylcarbonyl group represented by $R^{15}CO$- as said R1 or R2 include a linear or branched-chain alkylcarbonyl group having 2 to 18 carbon atoms (including the carbonyl carbon) (acetyl, propionyl, butanoyl, 2-methylpropionyl, heptanoyl, 2-methylbutanoyl, 3-methylbutanoyl, octanoyl, decanoyl, dodecanoyl, octadecanoyl or the like) and the like. Examples of the arylcarbonyl group include an arylcarbonyl group having 7 to 11 carbon atoms (including the carbonyl carbon) (benzoyl, naphthoyl or the like) and the like.

Examples of the carbonyloxy group represented by $R^{16}COO$- as said R1 or R2 include a linear or branched-chain acyloxy group having 2 to 19 carbon atoms (acetoxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, hexylcarbonyloxy, 2-ethylhexylcarbonyloxy, octylcarbonyloxy, tetradecylcarbonyloxy, octadecylcarbonyloxy or the like) and the like, an arylcarbonyloxy group having 6 to 18 carbon atoms (benzoyloxy, naphthoyloxy, pentafluorobenzoyloxy or the like) and the like, a carbonyloxy group bearing a linear or branched fluoroalkyl group having 1 to 8 carbon atoms (such as a trifluoromethyl, 2,2-difluoroethyl, 2,2,2-trifluoroethyl, pentafluoroethyl, 2,2,3,3-tetrafluoropropyl, 2,2,3,3,3-pentafluoropropyl, 1,1,1,3,3,3-hexafluoro-2-propyl, heptafluoropropyl, 2,2,3,3,4,4,4-heptafluorobutyl, perfluorobutyl, nonafluoro-tert-butyl, 1H,1H-nonafluoropent perfluoropentyl, 1H,1H-tridecafluorohexyl, perfluorohexyl, 1H,1H-pentadecafluorooctyl and perfluorooctyl group) or the like as $R^{16}$, and the like.

Examples of the oxycarbonyl group represented by $R^{17}OCO$- as said R1 or R2 include a linear or branched-chain alkoxycarbonyl group having 2 to 19 carbon atoms (including the carbonyl carbon) (methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, octyloxycarbonyl, tetradecyloxycarbonyl, octadecyloxycarbonyl or the like), an aryloxycarbonyl group having 7 to 11 carbon atoms (including the carbonyl carbon) (phenoxycarbonyl, naphthoxycarbonyl or the like) and the like.

Examples of $R^{18}$ of the carbonate group represented by $R^{18}OCOO$- as said R1 or R2 include a linear alkyl group having 1 to 18 carbon atoms (methyl, ethyl, n-propyl, n-butyl, n-pentyl, n-octyl, n-decyl, n-dodecyl, n-tetradecyl, n-hexadecyl, n-octadecyl or the like), a branched-chain alkyl group having 1 to 18 carbon atoms (isopropyl, isobutyl, sec-butyl, tert-butyl, isopentyl, neopentyl, tert-pentyl, isohexyl and isooctadecyl), a cycloalkyl group having 3 to 18 carbon atoms (cyclopropyl, cyclobutyl, cyclopentyl, cyclohexyl, 4-decylcyclohexyl or the like), a linear or branched fluoroalkyl group having 1 to 8 carbon atoms (such as a trifluoromethyl, 2,2-difluoroethyl, 2,2,2-trifluoroethyl, pentafluoroethyl, 2,2,3,3-tetrafluoropropyl, 2,2,3,3,3-pentafluoropropyl, 1,1,1,3,3,3-hexafluoro-2-propyl, heptafluoropropyl, 2,2,3,3,4,4,4-heptafluorobutyl, perfluorobutyl, nonafluoro-tert-butyl, 1H,1H-nonafluoropentyl, perfluoropentyl, 1H,1H-tridecafluorohex perfluorohexyl, 1H,1H-pentadecafluorooc and perfluorooctyl group) and the like.

Examples of $R^{19}$ of the urethane group represented by $R^{19}NHCOO$- as said R1 or R2 include a linear alkyl group having 1 to 18 carbon atoms (methyl, ethyl, n-propyl, n-butyl, n-pentyl, n-octyl, n-decyl, n-dodecyl, n-tetradecyl, n-hexadecyl, n-octadecyl or the like), a branched-chain alkyl group having 1 to 18 carbon atoms (isopropyl, isobutyl, sec-butyl, tert-butyl, isopentyl, neopentyl, tert-pentyl, isohexyl and isooctadecyl), a cycloalkyl group having 3 to 18 carbon

atoms (cyclopropyl, cyclobutyl, cyclopentyl, cyclohexyl, 4-decylcyclohexyl or the like), a linear or branched fluoroalkyl group having 1 to 8 carbon atoms (such as a trifluoromethyl, 2,2-difluoroethyl, 2,2,2-trifluoroethyl, pentafluoroethyl, 2,2,3,3-tetrafluoropropyl, 2,2,3,3,3-pentafluoropropyl, 1,1,1,3,3,3-hexafluoro-2-propyl, heptafluoropropyl, 2,2,3,3,4,4,4-heptafluorobutyl, perfluorobutyl, nonafluoro-tert-butyl, 1H,1H-nonafluoropentyl, perfluoropentyl, 1H,1H-tridecafluorohex perfluorohexyl, 1H,1H-pentadecafluorooc and perfluorooctyl group) and the like.

Examples of the halogen atom include a fluorine atom, a chlorine atom, a bromine atom and an iodine atom, and from the viewpoint of the solubility to a solvent, preferred are a fluorine atom, a chlorine atom and a bromine atom, and more preferred are a fluorine atom and a chlorine atom.

It is preferred that at least any one of R1 and R2 be an alkyl group having 1 to 6 carbon atoms, a fluoroalkyl group having 1 to 6 carbon atoms, a silyl group, an alkoxy group or aryloxy group represented by $R^{11}O$-, an alkylthio group or arylthio group represented by $R^{12}S$-, or a carbonyloxy group represented by $R^{16}COO$-.

Each of the above-described alkyl group having 1 to 18 carbon atoms or the fluoroalkyl group having 1 to 18 carbon atoms, the alkenyl group having 2 to 18 carbon atoms, the alkynyl group having 2 to 18 carbon atoms, the aryl group having 6 to 18 carbon atoms, the silyl group, the alkoxy group or aryloxy group represented by $R^{11}O$-, the alkylthio group or arylthio group represented by $R^{12}S$-, the sulfinyl group represented by $R^{13}SO$-, the sulfonyl group represented by $R^{14}SO_2$-, the alkylcarbonyl group or arylcarbonyl group represented by $R^{15}CO$-, the carbonyloxy group represented by $R^{16}COO$-, the oxycarbonyl group represented by $R^{17}OCO$-, the carbonate group represented by $R^{18}OCOO$- and the urethane group represented by $R^{19}NHCOO$- may have a substituent (T). Examples of the substituent (T) include an alkyl group, a hydroxy group, an alkoxy group, an alkylcarbonyl group, an arylcarbonyl group, an alkoxycarbonyl group, an aryloxycarbonyl group, an arylthiocarbonyl group, an acyloxy group, an arylthio group, an alkylthio group, an aryl group, a heterocyclic hydrocarbon group, an aryloxy group, an alkylsulfinyl group, an arylsulfinyl group, an alkylsulfonyl group, an arylsulfonyl group and a halogen atom. One kind of the substituent (T) or two or more kinds thereof may be employed.

Examples of the alkyl group include a linear alkyl group having 1 to 18 carbon atoms (methyl, ethyl, n-propyl, n-butyl, n-pentyl, n-octyl, n-decyl, n-dodecyl, n-tetradecyl, n-hexadecyl, n-octadecyl or the like), a branched-chain alkyl group having 1 to 18 carbon atoms (isopropyl, isobutyl, sec-butyl, tert-butyl, isopentyl, neopentyl, tert-pentyl, isohexyl and isooctadecyl), a cycloalkyl group having 3 to 18 carbon atoms (cyclopropyl, cyclobutyl, cyclopentyl, cyclohexyl, 4-decylcyclohexyl or the like), a linear or branched fluoroalkyl group having 1 to 3 carbon atoms (such as a trifluoromethyl, pentafluoroethyl and heptafluorobutyl group) and the like.

Examples of the alkoxy group include a linear or branched-chain alkoxy group having 1 to 18 carbon atoms (methoxy, ethoxy, propoxy, isopropoxy, butoxy, isobutoxy, sec-butoxy, tert-butoxy, hexyloxy, decyloxy, dodecyloxy, octadecyloxy or the like) and the like.

Examples of the alkylcarbonyl group include a linear or branched-chain alkylcarbonyl group having 2 to 18 carbon atoms (including the carbonyl carbon) (acetyl, propionyl, butanoyl, 2-methylpropionyl, heptanoyl, 2-methylbutanoyl, 3-methylbutanoyl, octanoyl, decanoyl, dodecanoyl, octadecanoyl or the like) and the like.

Examples of the arylcarbonyl group include an arylcarbonyl group having 7 to 11 carbon atoms (including the carbonyl carbon) (benzoyl, naphthoyl or the like) and the like.

Examples of the alkoxycarbonyl group include a linear or branched-chain alkoxycarbonyl group having 2 to 19 carbon atoms (including the carbonyl carbon) (methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, octyloxycarbonyl, tetradecyloxycarbonyl, octadecyloxycarbonyl or the like) and the like.

Examples of the aryloxycarbonyl group include an aryloxycarbonyl group having 7 to 11 carbon atoms (including the carbonyl carbon) (phenoxycarbonyl, naphthoxycarbonyl or the like) and the like.

Examples of the arylthiocarbonyl group include an arylthiocarbonyl group having 7 to 11 carbon atoms (including the carbonyl carbon) (phenylthiocarbonyl, naphthoxythiocarbonyl or the like) and the like.

Examples of the acyloxy group include a linear or branched-chain acyloxy group having 2 to 19 carbon atoms (acetoxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, octylcarbonyloxy, tetradecylcarbonyloxy, octadecylcarbonyloxy or the like) and the like.

Examples of the arylthio group include an arylthio group having 6 to 20 carbon atoms (phenylthio, 2-methylphenylthio, 3-methylphenylthio, 4-methylphenylthio, 2-chlorophenylthio, 3-chlorophenylthio, 4-chlorophenylthio, 2-bromophenylthio, 3-bromophenylthio, 4-bromophenylthio, 2-fluorophenylthio, 3-fluorophenylthio, 4-fluorophenylthio, 2-hydroxyphenylthio, 4-hydroxyphenylthio, 2-methoxyphenylthio, 4-methoxyphenylthio, 1-naphthylthio, 2-naphthylthio, 4-[4-(phenylthio)benzoyl]phenylthio, 4-[4-(phenylthio)phenoxy]phenylthio, 4-[4-(phenylthio)phenyl]phenylthio, 4-(phenylthio)phenylthio, 4-benzoylphenylthio, 4-benzoyl-2-chlorophenylthio, 4-benzoyl-3-chlorophenylthio, 4-benzoyl-3-methylthiophenylthio, 4-benzoyl-2-methylthiophenylthio, 4-(4-methylthiobenzoyl)phenylthio, 4-(2-methylthiobenzoyl)phenylthio, 4-(p-methylbenzoyl)phenylthio, 4-(p-ethylbenzoyl)phenylthio, 4-(p-isopropylbenzoyl)phenylthio, 4-(p-tert-butylbenzoyl)phenylthio or the like) and the like.

Examples of the alkylthio group include a linear or branched-chain alkylthio group having 1 to 18 carbon atoms (methylthio, ethylthio, propylthio, isopropylthio, butylthio, isobutylthio, sec-butylthio, tert-butylthio, pentylthio, isopentylthio, neopentylthio, tert-pentylthio, octylthio, decylthio, dodecylthio, isooctadecylthio or the like) and the like.

Examples of the aryl group include an aryl group having 6 to 10 carbon atoms (phenyl, tolyl, dimethylphenyl, naphthyl or the like) and the like.

Examples of the heterocyclic hydrocarbon group include a heterocyclic hydrocarbon group having 4 to 20 carbon atoms (thienyl, furanyl, pyranyl, pyrrolyl, oxazolyl, thiazolyl, pyridyl, pyrimidyl, pyrazinyl, indolyl, benzofuranyl, benzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl, dibenzofuranyl or the like) and the like.

Examples of the aryloxy group include an aryloxy group having 6 to 10 carbon atoms (phenoxy, naphthyloxy or the like) and the like.

Examples of the alkylsulfinyl group include a linear or branched-chain alkylsulfinyl group having 1 to 18 carbon atoms (methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, tert-pentylsulfinyl, octylsulfinyl, isooctadecylsulfinyl or the like) and the like.

Examples of the arylsulfinyl group include an arylsulfinyl group having 6 to 10 carbon atoms (phenylsulfinyl, tolylsulfinyl, naphthylsulfinyl or the like) and the like.

Examples of the alkylsulfonyl group include a linear or branched-chain alkylsulfonyl group having 1 to 18 carbon atoms (methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, tert-pentylsulfonyl, octylsulfonyl, octadecylsulfonyl or the like) and the like.

Examples of the arylsulfonyl group include an arylsulfonyl group having 6 to 10 carbon atoms {phenylsulfonyl, tolylsulfonyl (a tosyl group), naphthylsulfonyl or the like} and the like.

Examples of the halogen atom include a fluorine atom, a chlorine atom, a bromine atom, an iodine atom and the like.

Among these substituents (T), from the viewpoints of the ease of synthesis, the absorption wavelength region and the heat-resistant stability, preferred are the alkyl group, the alkoxy group, the arylcarbonyl group, the aryloxycarbonyl group, the arylthio group, the aryl group, the aryloxy group, the arylsulfinyl group, the arylsulfonyl group, the fluorine atom and the chlorine atom, and especially preferred are the methyl group, the tert-butyl group, the trifluoromethyl group, the fluorine and chlorine atoms.

It is preferred that the total number (m + n) of R1s and R2s lie within the range of 1 to 2, and it is further preferred that the equations of m = 1 and n = 0 or the equations of m = 1 and n = 1 are satisfied.

When the equations of m = 1 and n = 0 are satisfied, it is preferred that the position where R1 is bonded to the naphthalene ring be the 3-position or the 4-position. As R1 bonded to the 3-position, preferred are an alkoxy group or aryloxy group represented by $R^{11}O$- and a carbonyloxy group represented by $R^{16}COO$-, and especially preferred is a carbonyloxy group represented by $R^{16}COO$-. As R1 bonded to the 4-position, preferred is an alkyl group having 1 to 18 carbon atoms, a linear or branched fluoroalkyl group having 1 to 8 carbon atoms, a silyl group or an alkoxy group or aryloxy group represented by $R^{11}O$-, and especially preferred is a linear or branched fluoroalkyl group having 1 to 8 carbon atoms, a silyl group, an alkoxy group represented by $R^{11}O$- in which one part or all of hydrogen atoms of R11 are substituted by fluorine atoms, or an aryloxy group represented by $R^{11}O$- in which one part or all of hydrogen atoms are substituted by fluorine atoms.

When the equations of m = 1 and n = 1 are satisfied, it is preferred that the position where substituents are bonded to the naphthalene ring be the 3-position and the 6-position, it is further preferred that R1 and R2 be the same as each other, it is still further preferred that R1 and R2 be alkyl groups having 1 to 18 carbon atoms, and it is especially preferred that R1 and R2 be alkyl groups (such as a tert-butyl group, a neopentyl group and an adamantyl group) in which the carbon bonded to the naphthalene ring is a quaternary carbon.

R3 is a hydrocarbon group (one part or all of hydrogen atoms may be substituted by fluorine atoms) having 1 to 18 carbon atoms. The hydrocarbon group having 1 to 18 carbon atoms may have a substituent.

As the substituent, one exemplified as the substituent (T) can be used. Examples of the hydrocarbon group having 1 to 18 carbon atoms include an alkyl group, an aryl group and a heterocyclic hydrocarbon group.

Examples of the alkyl group include a linear alkyl group having 1 to 18 carbon atoms (methyl, ethyl, n-propyl, n-butyl, n-pentyl, n-octyl, n-decyl, n-dodecyl, n-tetradecyl, n-hexadecyl, n-octadecyl or the like), a branched-chain alkyl group having 1 to 18 carbon atoms (isopropyl, isobutyl, sec-butyl, tert-butyl, isopentyl, neopentyl, tert-pentyl, isohexyl and isooctadecyl), a cycloalkyl group having 3 to 18 carbon atoms (cyclopropyl, cyclobutyl, cyclopentyl, cyclohexyl, 4-decylcyclohexyl, 10-camphoryl or the like) and the like.

Examples of the aryl group include an aryl group having 6 to 10 carbon atoms (phenyl, tolyl, dimethylphenyl, naphthyl, pentafluorophenyl or the like) and the like.

Examples of the heterocyclic hydrocarbon group include a heterocyclic hydrocarbon group having 4 to 20 carbon atoms (thienyl, furanyl, pyranyl, pyrrolyl, oxazolyl, thiazolyl, pyridyl, pyrimidyl, pyrazinyl, indolyl, benzofuranyl, benzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl, dibenzofuranyl or the like) and the like.

Examples of a group in which one part or all of hydrogen atoms of the hydrocarbon group having 1 to 18 carbon atoms are substituted by fluorine atoms include CxFy having a large electron-attracting property.

CxFy having a large electron-attracting property is a functional group including 1 to 8 carbon atoms (x = 1 to 8) and 3 to 17 fluorine atoms (y = 3 to 17).

When the number of carbon atoms is 1 or more, a strong acid is easily synthesized, and when the number of carbon atoms is 8 or less, the non-ionic photoacid generator is excellent in heat-resistant stability. When the number of fluorine atoms is 3 or more, the acid generated is capable of acting as a strong acid, and when the number of fluorine atoms is 17 or less, a strong acid is easily synthesized.

One kind of CxFy may be used and two or more kinds thereof may be combinedly used in the non-ionic photoacid generator (A).

Examples of CxFy include a linear alkyl group (RF1), a branched-chain alkyl group (RF2), a cycloalkyl group (RF3) and an aryl group (RF4) which allow hydrogen atoms to be substituted by fluorine atoms.

Examples of the linear alkyl group (RF1) which allows hydrogen atoms to be substituted by fluorine atoms include a trifluoromethyl group (x = 1, y = 3), a pentafluoroethyl group (x = 2, y = 5), a nonafluorobutyl group (x = 4, y = 9), a perfluorohexyl group (x = 6, y = 13), a perfluorooctyl group (x = 8, y = 17) and the like.

Examples of the branched-chain alkyl group (RF2) which allows hydrogen atoms to be substituted by fluorine atoms include a perfluoroisopropyl group (x = 3, y = 7), a perfluoro-tert-butyl group (x = 4, y = 9), a perfluoro-2-ethylhexyl group (x = 8, y = 17) and the like.

Examples of the cycloalkyl group (RF3) which allows hydrogen atoms to be substituted by fluorine atoms include a perfluorocyclobutyl group (x = 4, y = 7), a perfluorocyclopentyl group (x = 5, y = 9), a perfluorocyclohexyl group (x = 6, y = 11), a perfluoro(1-cyclohexyl)methyl group (x = 7, y = 13) and the like.

Examples of the aryl group (RF4) which allows hydrogen atoms to be substituted by fluorine atoms include a pentafluorophenyl group (x = 6, y = 5), a 3-trifluoromethyltetrafluorophenyl group (x = 7, y = 7) and the like.

Among R3s, preferred are the linear alkyl group having 1 to 18 carbon atoms, the branched-chain alkyl group having 1 to 18 carbon atoms, the cycloalkyl group having 3 to 18 carbon atoms and CxFy, and further preferred is CxFy having a large electron-attracting property.

Among CxFys having a large electron-attracting property, from the viewpoints of easy availability and the decomposition property of the sulfonic acid ester moiety, preferred are a linear alkyl group (RF1), a branched-chain alkyl group (RF2) and an aryl group (RF4), further preferred are a linear alkyl group (RF1) and an aryl group (RF4), and especially preferred are a trifluoromethyl group (x = 1, y = 3), a pentafluoroethyl group (x = 2, y = 5), a heptafluoropropyl group (x = 3, y = 7), a nonafluorobutyl group (x = 4, y = 9) and a pentafluorophenyl group (x = 6, y = 5).

Although the synthetic method of the non-ionic photoacid generator (A) according to the present invention is not particularly limited as long as the aimed product is synthesized, for example, the non-ionic photoacid generator can be synthesized by the reaction of an N-hydroxyimide compound (P1) as a precursor and a sulfonic acid anhydride shown by $(R3\text{-}SO_2)_2O$ or the reaction of a salt of an N-hydroxyimide compound (P1) and sulfonic acid chloride shown by $R3\text{-}SO_2Cl$.

The non-ionic photoacid generator (A) according to the present invention may be previously dissolved in a solvent not inhibiting the reaction in order to facilitate the dissolution thereof in a resist material.

Examples of the solvent include a carbonate (propylene carbonate, ethylene carbonate, 1,2-butylene carbonate, dimethyl carbonate, diethyl carbonate, and the like); an ester (ethyl acetate, ethyl lactate, β-propiolactone, β-butyrolactone, γ-butyrolactone, δ-valerolactone, ε-caprolactone and the like); an ether (ethylene glycol monomethyl ether, propylene glycol monoethyl ether, diethylene glycol monombutyl ether, dipropylene glycol dimethyl ether, triethylene glycol diethyl ether, tripropylene glycol dibutyl ether, and the like); an ether ester (ethylene glycol monomethyl ether acetate, propylene glycol monoethyl ether acetate, diethylene glycol monombutyl ether acetate, and the like) and the like.

In the case of using a solvent, the proportion of the solvent used is preferably 15 to 1000 parts by weight, and further preferably 30 to 500 parts by weight, relative to 100 parts by weight of the photoacid generator according to the present invention.

Since the resin composition (Q) for photolithography according to the present invention includes the non-ionic photoacid generator (A) as an essential component, by performing ultraviolet ray irradiation and post exposure baking (PEB), a difference in solubility against a developing solution between the exposed part and the unexposed part is generated. One kind of the non-ionic photoacid generator (A) can be used alone or two or more kinds thereof can be used in combination.

Examples of the resin composition (Q) for photolithography include a mixture of a negative type chemical

amplification resin (QN) and a non-ionic photoacid generator (A); and a mixture of a positive type chemical amplification resin (QP) and a non-ionic photoacid generator (A).

The negative type chemical amplification resin (QN) may be constituted of a phenolic hydroxyl group-containing resin (QN1) and a crosslinking agent (QN2).

No particular restriction is put on the phenolic hydroxyl group-containing resin (QN1) as long as the resin is a resin containing a phenolic hydroxyl group, and for example, a novolak resin, polyhydroxystyrene, a copolymer of hydroxystyrene, a copolymer of hydroxystyrene and styrene, a copolymer of hydroxystyrene, styrene and a (meth)acrylic acid derivative, a phenol-xylylene glycol condensation resin, a cresol-xylylene glycol condensation resin, a polyimide containing a phenolic hydroxyl group, a polyamic acid containing a phenolic hydroxyl group, a phenol-dicyclopentadiene condensation resin, and the like can be used. Of these, preferred are a novolak resin, polyhydroxystyrene, a copolymer of polyhydroxystyrene, a copolymer of hydroxystyrene and styrene, a copolymer of hydroxystyrene, styrene and a (meth)acrylic acid derivative, and a phenol-xylylene glycol condensation resin. In this connection, one kind of these phenolic hydroxyl group-containing resins (QN1) may be used alone and two or more kinds thereof may be mixedly used.

For example, the above-described novolak resin can be obtained by allowing a kind of phenol and a kind of aldehyde to undergo a condensation in the presence of a catalyst.

Examples of the above-described kind of phenol include phenol, o-cresol, m-cresol, p-cresol, o-ethylphenol, m-ethylphenol, p-ethylphenol, o-butylphenol, m-butylphenol, p-butylphenol, 2,3-xylenol, 2,4-xylenol, 2,5-xylenol, 2,6-xylenol, 3,4-xylenol, 3,5-xylenol, 2,3,5-trimethylphenol, 3,4,5-trimethylphenol, catechol, resorcinol, pyrogallol, $\alpha$-naphthol, $\beta$-naphthol and the like.

Moreover, examples of the above-described kind of aldehyde include formaldehyde, paraformaldehyde, acetaldehyde, benzaldehyde and the like.

Specific examples of the novolak resin include a phenol/formaldehyde condensation novolak resin, a cresol/formaldehyde condensation novolak resin, a phenol-naphthol/formaldehyde condensation novolak resin, and the like.

Moreover, in the above-described phenolic hydroxyl group-containing resin (QN1), a phenolic low-molecular compound may be contained as one part of the components.

Examples of the above-described phenolic low-molecular compound include 4,4'-dihydroxydiphenylmethane, 4,4'-dihydroxydiphenylether, tris(4-hydroxyphenyl)methane, 1,1-bis(4-hydroxyphenyl)-1-phenylethane, tris(4-hydroxyphenyl)ethane, 1,3-bis[1-(4-hydroxyphenyl)-1-methylethyl]benzene, 1,4-bis[1-(4-hydroxyphenyl)-1-methylethyl]benzene, 4,6-bis[1-(4-hydroxyphenyl)-1-methylethyl]-1,3-dihydroxybenzene, 1,1-bis(4-hydroxyphenyl)-1-[4-[1-(4-hydroxyphenyl)-1-methylethyl]phenyl]ethane, 1,1,2,2-tetra(4-hydroxyphenyl)ethane, 4,4'-{1-[4-[1-(4-hydroxyphenyl)-1-methylethyl]phenyl]ethylidene}bisphenol, and the like. One kind of these phenolic low-molecular compounds may be used alone and two or more kinds thereof may be mixedly used.

The proportion of this phenolic low-molecular compound contained in the phenolic hydroxyl group-containing resin (QN1) is preferably 40% by weight or less, and more preferably 1 to 30% by weight, in the case where the amount of the phenolic hydroxyl group-containing resin (QN1) is defined as 100% by weight.

From the viewpoints of the resolution characteristics, thermal shock resistance, heat resistance, residual film ratio, and the like of the resulting insulation film, the weight-average molecular weight of the phenolic hydroxyl group-containing resin (QN1) is preferably 2000 or more, and more preferably 2000 to 20000 or so.

Moreover, the proportion of the phenolic hydroxyl group-containing resin (QN1) contained in a negative type chemical amplification resin (QN) is preferably 30 to 90% by weight, and more preferably 40 to 80% by weight, in the case where the whole composition excluding the solvent is defined as 100% by weight. In the case where the proportion of this phenolic hydroxyl group-containing resin (QN1) contained is 30 to 90% by weight, it is preferred because a film formed with a photosensitive insulation resin composition has a sufficient developability in an aqueous alkali solution.

The crosslinking agent (QN2) is not particularly limited as long as the crosslinking agent is a compound capable of allowing the phenolic hydroxyl group-containing resin (QN1) to be crosslinked by a strong acid generated from the non-ionic photoacid generator (A).

Examples of the crosslinking agent (QN2) include a bisphenol A-based epoxy compound, a bisphenol F-based epoxy compound, a bisphenol S-based epoxy compound, a novolak resin-based epoxy compound, a resole resin-based epoxy compound, a poly(hydroxystyrene)-based epoxy compound, an oxetane compound, a methylol group-containing melamine compound, a methylol group-containing benzoguanamine compound, a methylol group-containing urea compound, a methylol group-containing phenol compound, an alkoxyalkyl group-containing melamine compound, an alkoxyalkyl group-containing benzoguanamine compound, an alkoxyalkyl group-containing urea compound, an alkoxyalkyl group-containing phenol compound, a carboxymethyl group-containing melamine resin, a carboxymethyl group-containing benzoguanamine resin, a carboxymethyl group-containing urea resin, a carboxymethyl group-containing phenol resin, a carboxymethyl group-containing melamine compound, a carboxymethyl group-containing benzoguanamine compound, a carboxymethyl group-containing urea compound, a carboxymethyl group-containing phenol compound, and the like.

Among these crosslinking agents (QN2), preferred are a methylol group-containing phenol compound, a methoxymethyl group-containing melamine compound, a methoxymethyl group-containing phenol compound, a methoxymethyl group-containing glycoluril compound, a methoxymethyl group-containing urea compound and an acetoxymethyl group-containing phenol compound, and further preferred are a methoxymethyl group-containing melamine compound (for example, hexamethoxymethylmelamine or the like), a methoxymethyl group-containing glycoluril compound, a methoxymethyl group-containing urea compound, and the like. The methoxymethyl group-containing melamine compound is commercially available under the trade name of CYMEL 300, CYMEL 301, CYMEL 303, CYMEL 305 (available from MITSUI CYANAMID Co., Ltd.) or the like, the methoxymethyl group-containing glycoluril compound is commercially available under the trade name of CYMEL 1174 (available from MITSUI CYANAMID Co., Ltd.) or the like, and moreover, the methoxymethyl group-containing urea compound is commercially available under the trade name of MX290 (available from SANWA CHEMICAL CO., LTD.) or the like.

From the viewpoints of the lowering in the residual film ratio, the pattern meandering, the pattern swelling and the developability, the content of the crosslinking agent (QN2) is usually 5 to 60% by mole, preferably 10 to 50% by mole, and further preferably 15 to 40% by mole, relative to the whole amount of acidic functional groups in the phenolic hydroxyl group-containing resin (QN1).

Examples of the positive type chemical amplification resin (QP) include a protecting group-introduced resin (QP2) prepared by allowing one part or all of hydrogen atoms of the acidic functional group in an alkali-soluble resin (QP1) containing one kind or more of acidic functional groups such as a phenolic hydroxyl group, a carboxyl group, and a sulfonyl group to be substituted by acid-dissociable groups.

In this connection, the acid-dissociable group is a group capable of being dissociated in the presence of a strong acid generated from the non-ionic photoacid generator (A).

The protecting group-introduced resin (QP2) is alkali-insoluble or hardly alkali-soluble in itself.

Examples of the alkali-soluble resin (QP1) include a phenolic hydroxyl group-containing resin (QP11), a carboxyl group-containing resin (QP12), a sulfonic acid group-containing resin (QP13) and the like.

As the phenolic hydroxyl group-containing resin (QP11), one that is the same as the above-described phenolic hydroxyl group-containing resin (QN1) can be used.

No particular restriction is put on the carboxyl group-containing resin (QP12) as long as the resin is a polymer having a carboxyl group, and for example, the resin can be obtained by allowing a carboxyl group-containing vinyl monomer (Ba) and an optional hydrophobic group-containing vinyl monomer (Bb) to undergo a vinyl polymerization.

Examples of the carboxyl group-containing vinyl monomer (Ba) include an unsaturated monocarboxylic acid [such as (meth)acrylic acid, crotonic acid and cinnamic acid], an unsaturated polyvalent (di- to tetravalent) carboxylic acid [such as maleic acid (maleic anhydride), itaconic acid, fumaric acid and citraconic acid], an unsaturated polycarboxylic acid alkyl (an alkyl group with 1 to 10 carbon atoms) ester [such as a maleic acid monoalkyl ester, a fumaric acid monoalkyl ester and a citraconic acid monoalkyl ester], and a salt thereof [such as an alkali metal salt (a sodium salt, a potassium salt and the like), an alkaline earth metal salt (a calcium salt, a magnesium salt and the like), an amine salt and an ammonium salt].

Among these, from the viewpoints of the polymerizability and easy availability, preferred is an unsaturated monocarboxylic acid, and further preferred is a (meth)acrylic acid.

Examples of the hydrophobic group-containing vinyl monomer (Bb) include a (meth)acrylic acid ester (Bb1), an aromatic hydrocarbon monomer (Bb2) and the like.

Examples of the (meth)acrylic acid ester (Bb1) include an alkyl (meth)acrylate bearing an alkyl group with 1 to 20 carbon atoms [such as methyl (meth)acrylate, ethyl (meth)acrylate, n-propyl (meth)acrylate, isopropyl

(meth)acrylate, n-butyl (meth)acrylate, n-hexyl (meth)acrylate and 2-ethylhexyl (meth)acrylate], an alicyclic group-containing (meth)acrylate [such as dicyclopentanyl (meth)acrylate, dicyclopentenyl (meth)acrylate and isobornyl (meth)acrylate] and the like.

Examples of the aromatic hydrocarbon monomer (Bb2) include a hydrocarbon monomer having a styrene skeleton [such as styrene, $\alpha$-methylstyrene, vinyltoluene, 2,4-dimethylstyrene, ethylstyrene, isopropylstyrene, butylstyrene, phenylstyrene, cyclohexylstyrene and benzylstyrene], vinylnaphthalene and the like.

The charged monomer mole ratio of (Ba)/(Bb) in the carboxyl group-containing resin (QP12) is usually 10 to 100/0 to 90, from the viewpoint of the developability, preferably 10 to 80/20 to 90, and further preferably 25 to 85/15 to 75.

No particular restriction is put on the sulfonic acid group-containing resin (QP13) as long as the resin is a polymer having a sulfonic acid group, and for example, the resin can be obtained by allowing a sulfonic acid group-containing vinyl monomer (Bc) and an optional hydrophobic group-containing vinyl monomer (Bb) to undergo a vinyl polymerization.

As the hydrophobic group-containing vinyl monomer (Bb), one that is the same as the above-described one can be used.

Examples of the sulfonic acid group-containing vinyl monomer (Bc) include vinylsulfonic acid, (meth)allylsulfonic acid, styrenesulfonic acid, $\alpha$-methylstyrenesulfonic acid, 2-(meth)acryloylamido-2-methylpropane sulfonic acid, and a salt thereof. Examples of the salt include an alkali metal (sodium, potassium or the like) salt, an alkaline earth metal

(calcium, magnesium or the like) salt, a primary to tertiary amine salt, an ammonium salt, a quaternary ammonium salt, and the like.

The charged monomer mole ratio of (Bc)/(Bb) in the sulfonic acid group-containing resin (QP13) is usually 10 to 100/0 to 90, from the viewpoint of the developability, preferably 10 to 80/20 to 90, and further preferably 25 to 85/15 to 75.

With regard to the HLB value of the alkali-soluble resin (QP1), although the preferred range varies depending on the resin skeleton of the alkali-soluble resin (QP1), the HLB value preferably lies within the range of 4 to 19, further preferably lies within the range of 5 to 18, and especially preferably lies within the range of 6 to 17.

When the HLB value lies within the range of 4 or more, the developability is further improved at the time of performing developing, and when the HLB value lies within the range of 19 or less, the water resistance of a cured material is further improved.

In this connection, the HLB in the present invention refers to an HLB value according to the Oda method and refers to the hydrophilicity-hydrophobicity balance value, and the HLB can be calculated from the ratio of the organicity value and the inorganicity value of an organic compound.

$$\mathrm{HLB} \approx 10 \times \mathrm{Inorganicity/Organicity}$$

Moreover, the inorganicity value and the organicity value are described in detail in the document "Kaimen kasseizai no gosei to sono oyo" (published by MAKI SYOTEN, written by ODA, TERAMURA) p. 501; or "Shin●Kaimen kasseizai nyumon" (Takehiko FUJIMOTO, published by Sanyo Chemical Industries, Ltd.) p. 198.

For example, the acid-dissociable group in the protecting group-introduced resin (QP2) can be exemplified by a substituted methyl group, a 1-substituted ethyl group, a 1-branched alkyl group, a silyl group, a germyl group, an alkoxycarbonyl group, an acyl group, a cyclic acid-dissociable group, or the like. One kind of these may be used alone, and two or more kinds thereof may be used in combination.

For example, the 1-substituted methyl group can be exemplified by a methoxymethyl group, a methylthiomethyl group, an ethoxymethyl group, an ethylthiomethyl group, a methoxyethoxymethyl group, a benzyloxymethyl group, a benzylthiomethyl group, a phenacyl group, a bromophenacyl group, a methoxyphenacyl group, a methylthiophenacyl group, an α-methylphenacyl group, a cyclopropylmethyl group, a benzyl group, a diphenylmethyl group, a triphenylmethyl group, a bromobenzyl group, a nitrobenzyl group, a methoxybenzyl group, a methylthiobenzyl group, an ethoxybenzyl group, an ethylthiobenzyl group, a piperonyl group, a methoxycarbonylmethyl group, an ethoxycarbonylmethyl group, an n-propoxycarbonylmethyl group, an i-propoxycarbonylmethyl group, an n-butoxycarbonylmethyl group, a tert-butoxycarbonylmethyl group, or the like.

For example, the 1-substituted ethyl group can be exemplified by a 1-methoxyethyl group, a 1-methylthioethyl group, a 1,1-dimethoxyethyl group, a 1-ethoxyethyl group, a 1-ethylthioethyl group, a 1,1-diethoxyethyl group, a 1-ethoxypropyl group, a 1-propoxyethyl group, a 1-cyclohexyloxyethyl group, a 1-phenoxyethyl group, a 1-phenylthioethyl group, a 1,1-diphenoxyethyl group, a 1-benzyloxyethyl group, a 1-benzylthioethyl group, a 1-cyclopropylethyl group, a 1-phenylethyl group, a 1,1-diphenylethyl group, a 1-methoxycarbonylethyl group, a 1-ethoxycarbonylethyl group, a 1-n-propoxycarbonylethyl group, a 1-isopropoxycarbonylethyl group, a 1-n-butoxycarbonylethyl group, a 1-tert-butoxycarbonylethyl group, or the like.

For example, the 1-branched alkyl group can be exemplified by an i-propyl group, a sec-butyl group, a tert-butyl group, 1,1-dimethylpropyl group, 1-methylbutyl group, 1,1-dimethylbutyl group, or the like.

For example, the silyl group can be exemplified by a tricarbylsilyl group such as a trimethylsilyl group, an ethyldimethylsilyl group, a methyldiethylsilyl group, a triethylsilyl group, an i-propyldimethylsilyl group, a methyldi-i-propylsilyl group, a tri-i-propylsilyl group, a tert-butyldimethylsilyl group, a methyldi-tert-butylsilyl group, a tri-tert-butylsilyl group, a phenyldimethylsilyl group, a methyldiphenylsilyl group and a triphenylsilyl group.

For example, the germyl group can be exemplified by a tricarbylgermyl group such as a trimethylgermyl group, an ethyldimethylgermyl group, a methyldiethylgermyl group, a triethylgermyl group, an isopropyldimethylgermyl group, a methyldi-i-propylgermyl group, a tri-i-propylgermyl group, a tert-butyldimethylgermyl group, a methyldi-tert-butylgermyl group, a tri-tert-butylgermyl group, a phenyldimethylgermyl group, a methyldiphenylgermyl group and a triphenylgermyl group.

For example, the alkoxycarbonyl group can be exemplified by a methoxycarbonyl group, an ethoxycarbonyl group, an i-propoxycarbonyl group, a tert-butoxycarbonyl group, or the like.

For example, the acyl group can be exemplified by an acetyl group, a propionyl group, a butyryl group, a heptanoyl group, a hexanoyl group, a valeryl group, a pivaloyl group, an isovaleryl group, a lauroyl group, a myristoyl group, a palmitoyl group, a stearoyl group, an oxalyl group, a malonyl group, a succinyl group, a glutaryl group, an adipoyl group, a piperoyl group, a suberoyl group, an azelaoyl group, a sebacoyl group, an acryloyl group, a propioloyl group, a methacryloyl group, a crotonoyl group, an oleoyl group, a maleoyl group, a fumaroyl group, a mesaconoyl

group, a campholoyl group, a benzoyl group, a phthaloyl group, an isophthaloyl group, a terephthaloyl group, a naphthoyl group, a toluoyl group, a hydroatropoyl group, an atropoyl group, a cinnamoyl group, a furoyl group, a thenoyl group, a nicotinoyl group, an isonicotinoyl group, a p-toluenesulfonyl group, a mesyl group, or the like.

For example, the cyclic acid-dissociable group can be exemplified by a cyclopropyl group, a cyclopentyl group, a cyclohexyl group, a cyclohexenyl group, a 4-methoxycyclohexyl group, a tetrahydropyranyl group, a tetrahydrofuranyl group, a tetrahydrothiopyranyl group, a tetrahydrothiofuranyl group, a 3-buromotetrahydropyranyl group, a 4-methoxytetrahydropyranyl group, a 4-methoxytetrahydrothiopyranyl group, 3-tetrahydrothiophene-1,1-dioxide, or the like.

Among these acid-dissociable groups, preferred are a tert-butyl group, a benzyl group, a 1-methoxyethyl group, a 1-ethoxyethyl group, a trimethylsilyl group, a tert-butoxycarbonyl group, a tert-butoxycarbonylmethyl group, a tetrahydropyranyl group, a tetrahydrofuranyl group, a tetrahydrothiopyranyl group, a tetrahydrothiofuranyl group, and the like.

Although the introduction ratio of the acid-dissociable group in the protecting group-introduced resin (QP2) {the proportion of the number of acid-dissociable groups to the total number of nonprotected acidic functional groups and acid-dissociable groups in the protecting group-introduced resin (QP2)} cannot be decided sweepingly according to the kind of an acid-dissociable group or an alkali-soluble resin into which the group is introduced, the introduction ratio is preferably 10 to 100%, and further preferably 15 to 100%.

With regard to the protecting group-introduced resin (QP2), the weight-average molecular weight in terms of polystyrene (hereinafter, referred to as "Mw") measured by gel permeation chromatography (GPC) is preferably 1,000 to 150,000, and further preferably 3,000 to 100,000.

Moreover, with regard to the protecting group-introduced resin (QP2), the ratio of the Mw to the number-average molecular weight in terms of polystyrene (hereinafter, referred to as "Mn") measured by gel permeation chromatography (GPC) (Mw/Mn) is usually 1 to 10, and preferably 1 to 5.

The content of the non-ionic photoacid generator (A) based on the weight of the solid content of the resin composition (Q) for photolithography is preferably 0.001 to 20% by weight, further preferably 0.01 to 15% by weight, and especially preferably 0.05 to 7% by weight.

When the content is 0.001% by weight or more, the sensitivity to ultraviolet rays can be further satisfactorily exhibited, and when the content is 20% by weight or less, the physical properties of a part insoluble to an alkali developing solution can be further satisfactorily exhibited.

For example, a resist prepared with the resin composition (Q) for photolithography according to the present invention can be formed by applying a resin solution prepared by dissolving the resin in a prescribed organic solvent (by dissolving the resin and dispersing inorganic fine particles in the case where the inorganic fine particles are included) on a substrate using a known method such as spin coating, curtain coating, roll coating, spray coating and screen printing, and then, allowing the solvent to be dried by heating or hot air blasting.

The organic solvent allowing the resin composition (Q) for photolithography to be dissolved therein is not particularly limited as long as the solvent is one capable of dissolving the resin composition therein and adjusting the physical properties (viscosity and the like) of the resin solution within an applicable range for spin coating or the like. For example, a known solvent such as N-methylpyrrolidone, N,N-dimethylformamide, dimethyl sulfoxide, toluene, ethanol, cyclohexanone, methanol, methyl ethyl ketone, ethyl acetate, butyl acetate, ethyl lactate, propylene glycol monomethyl ether acetate, acetone and xylene can be used.

Among these solvents, from the viewpoints of the drying temperature and the like, preferred is one with a boiling point of 200°C or lower (toluene, ethanol, cyclohexanone, methanol, methyl ethyl ketone, ethyl acetate, butyl acetate, ethyl lactate, propylene glycol monomethyl ether acetate, acetone and xylene), and moreover, these solvents can be used alone or in combination of two or more kinds thereof.

In the case of using an organic solvent, although the amount of the solvent blended is not particularly limited, the amount thereof blended is usually preferably 30 to 1,000% by weight, further preferably 40 to 900% by weight, and especially preferably 50 to 800% by weight, on the basis of the weight of the solid content of the resin composition (Q) for photolithography.

Although the drying condition for the resin solution after being applied varies with the kind of a solvent to be used, the drying is performed preferably within a range of 50 to 200°C and a range of 2 to 30 minutes, and the drying condition is appropriately determined in view of the amount (% by weight) of a residual solvent in the resin composition (Q) for photolithography after being dried, and the like.

A resist is formed on a substrate, after which photoirradiation corresponding to a wiring pattern shape is performed. Afterward, post exposure baking (PEB) is performed, and then, alkali development is performed to form the wiring pattern.

Examples of a method for performing photoirradiation include a method of performing exposure of a resist by means of an active light beam via a photomask having a wiring pattern. No particular restriction is put on the active light beam used for photoirradiation as long as the non-ionic photoacid generator (A) in the resin composition (Q) for photolithography according to the present invention can be decomposed.

Examples of the active light beam include a low pressure mercury lamp, a middle pressure mercury lamp, a

high pressure mercury lamp, an ultra-high pressure mercury lamp, a xenon lamp, a metal halogen lamp, an electron beam irradiation device, an X-ray irradiation device, a laser (an argon laser, a dye laser, a nitrogen laser, an LED, a helium-cadmium laser, or the like) and the like. Among these, preferred are a high pressure mercury lamp and an ultra-high pressure mercury lamp.

The temperature at the time of post exposure baking (PEB) is usually 40 to 200°C, preferably 50 to 190°C, and further preferably 60 to 180°C. When the temperature is lower than 40°C, since the deprotective reaction or the crosslinking reaction does not proceed sufficiently, the difference in solubility between the ultraviolet ray-irradiated part and the ultraviolet ray-unirradiated part is too small to form a pattern, and when the temperature is higher than 200°C, there is a problem that the productivity is lowered.

The heating time is usually 0.5 to 120 minutes, and is preferably 1 to 90 minutes and further preferably 2 to 90 minutes. When the time is shorter than 0.5 minute, it is difficult to control the time and the temperature, and when the time is longer than 120 minutes, there is a problem that the productivity is lowered.

Examples of a method for performing alkali development include a method of removing a part to be dissolved by means of an alkali developing solution so as to form a wiring pattern. No particular restriction is put on the alkali developing solution as long as a condition of creating a difference in solubility between the ultraviolet ray-irradiated part and the ultraviolet ray-unirradiated part of the resin composition (Q) for photolithography can be attained.

Examples of the alkali developing solution include an aqueous sodium hydroxide solution, an aqueous potassium hydroxide solution, an aqueous solution of sodium bicarbonate and a tetramethylammonium salt, and the like.

These alkali developing solutions may be added with a water-soluble organic solvent. Examples of the water-soluble organic solvent include methanol, ethanol, isopropyl alcohol, tetrahydrofuran, N-methylpyrrolidone and the like.

Although examples of a developing method include a dip system, a shower system and a spray system using an alkali developing solution, more preferred is a spray system.

With regard to the temperature of a developing solution, the developing solution is used preferably at 25 to 40°C. The developing time is appropriately determined according to the thickness of a resist.

## EXAMPLES

Hereinafter, the present invention will be further described by reference to examples and comparative examples, but the present invention should not be limited thereto. Hereinafter, % refers to % by weight and a part refers to a part by weight unless otherwise specified.

### Production Example 1

Synthesis of N-(n-propyl)-4-chloro-1,8-naphthalimide [Intermediate (1)]

In DMF (100 mL), 4-chloro-1,8-naphthalic anhydride (23.3 g, 100 mmol) was dissolved, and to this, n-propylamine (5.6 g, 110 mmol) and pyridine (0.1 g, 1 mmol) were added to allow the contents to undergo a reaction for 3 hours at 90°C. Then, the reaction liquid was added in small portions to stirred water (500 mL) contained in a beaker, after which the precipitated yellow solid matter was filtered and washed with water to obtain 20.5 g of the title compound [Intermediate (1)]. The product was identified by $^1$H-NMR. {$^1$H-NMR: 300MHz, DMSO-d6, $\delta$(ppm): 8.5(m, 2H), 8.4(d, 1H), 7.9(m, 2H), 4.0(t, 2H), 1.6(m, 2H), 0.9 (t, 3H)}

### Production Example 2

Synthesis of 4-isopropoxy-1,8-naphthalic anhydride

[Intermediate (2)]

In isopropyl alcohol (100 mL), Intermediate (1) (1.2 g, 4.3 mmol) obtained in Production Example 1 was dissolved, and to this solution, sodium hydride (10 g, 43 mmol) was added in small portions to allow the contents to undergo a reaction for 15 hours at 80°C. To this, water (100 mL) and potassium hydroxide (30 g) were added to further allow the contents to undergo a reaction for 10 hours at 60°C. This reaction liquid was cooled to room temperature (25°C), the reaction liquid was added in small portions to stirred water (500 mL) contained in a beaker, furthermore, hydrochloric acid was added thereto until the liquid becomes acidic, and the precipitated dark reddish-purple solid matter was filtered. This dark reddish-purple solid matter was recrystallized from isobutanol to obtain the title compound [Intermediate (2)]. The product was identified by $^1$H-NMR. {$^1$H-NMR: 300MHz, DMSO-d6, $\delta$(ppm)8.6-8.4(m, 3H), 7.8 (t, 1H), 7.3(d, 1H), 5.1(m, 1H), 1.4(d, 6H)}

Production Example 3

Synthesis of 4-n-propoxy-1,8-naphthalic anhydride

[Intermediate (3)]

The title compound [Intermediate (3)] was obtained in the same manner as that in Production Example 2 except that the isopropyl alcohol (100 mL) was changed to n-propyl alcohol (100 mL). The product was identified by $^{1}$H-NMR. {$^{1}$H-NMR: 300MHz, DMSO-d6, δ(ppm)8.6-8.4(m, 3H), 7.8(t, 1H), 7.3(d, 1H), 3.7(t, 2H), 1.6(m, 2H), 0.9(t, 3H)}

Production Example 4

Synthesis of 4-(2,2,3,3-tetrafluoro-1-propoxy)-1,8-naphthalic anhydride [Intermediate (4)]

The title compound [Intermediate (4)] was obtained in the same manner as that in Production Example 2 except that the isopropyl alcohol (100 mL) was changed to 2,2,3,3-tetrafluoro-1-propanol (100 mL). The product was identified by $^{1}$H-NMR and $^{19}$F-NMR. {$^{1}$H-NMR: 300MHz, DMSO-d6, δ(ppm)8.6-8.4(m, 3H), 7.8(t, 1H), 7.3(d, 1H), 5.9(tt, 1H), 3.8 (t, 2H), $^{19}$F-NMR: 300MHz, DMSO-d6, δ(ppm) -122 (d, 2F), - 133 (d, 2F)}

Production Example 5

Synthesis of 4-phenylacetyl-1,8-naphthalic anhydride

[Intermediate (5)]

In DMF (60 mL), 4-bromo-1,8-naphthalic anhydride (4.1 g, 15 mmol), phenylacetylene (1.5 g, 15 mmol) and triethylamine (3.0 g, 30 mmol) were dissolved, and furthermore, to this solution, bis(triphenylphosphine)palladium dichloride (0.1 g, 0.2 mmol) and copper iodide (0.03 g) were added to allow the contents to be stirred for 1 hour at 150°C under a nitrogen atmosphere. After being cooled to room temperature, the reaction liquid was charged into 1 N hydrochloric acid, and the precipitate was filtered and washed with water to obtain the title compound [Intermediate (5)]. The product was identified by $^{1}$H-NMR. {$^{1}$H-NMR: 300MHz, DMSO-d6, δ(ppm)8.6-8.4(m, 5H), 7.9-7.7(m, 3H), 7.3(d, 1H), 7.1(t, 1H)}

Production Example 6

Synthesis of 4-pentafluorophenoxy-1,8-naphthalic anhydride

[Intermediate (6)]

In N-methylpyrrolidone (50 mL), 4-bromo-1,8-naphthalic anhydride (4.1 g, 15 mmol) and pentafluorophenol (2.9 g, 16 mmol) were dissolved, and to this solution, sodium hydroxide (0.5 g, 1.6 mmol) and copper iodide (0.03 g) were added to allow the contents to be stirred for 5 hours at 150°C under a nitrogen atmosphere. After being cooled to room temperature, the reaction liquid was charged into 1 N hydrochloric acid, and the precipitate was filtered and washed with water to obtain the title compound [Intermediate (6)]. The product was identified by $^{1}$H-NMR and $^{19}$F-NMR. {$^{1}$H-NMR: 300MHz, DMSO-d6, δ (ppm) 8.6-8.4 (m, 3H), 7.8(d, 1H), 7.1(t, 1H), $^{19}$F-NMR: 300MHz, DMSO-d6, δ (ppm) -146 (d, 2F), -149 (t, 1F), -155 (t, 2F)}

Production Example 7

Synthesis of 4-(3-trifluoromethylphenoxy)-1,8-naphthalic anhydride [Intermediate (7)]

In N-methylpyrrolidone (50 mL), 4-bromo-1,8-naphthalic anhydride (4.1 g, 15 mmol) and 3-trifluoromethylphenol (2.9 g, 16 mmol) were dissolved, and to this solution, sodium hydroxide (0.5 g, 1.6 mmol) and copper iodide (0.03 g) were added to allow the contents to be stirred for 5 hours at 150°C under a nitrogen atmosphere. After being cooled to room temperature, the reaction liquid was charged into 1 N hydrochloric acid, and the precipitate was filtered and washed with water to obtain the title compound [Intermediate (7)]. The product was identified by $^{1}$H-NMR and $^{19}$F-NMR. {$^{1}$H-NMR: 300MHz, DMSO-d6, δ (ppm) 8.7-8.5(m, 3H), 7.9-7.6(m, 4H), 7.1-7.0(m, 2H), $^{19}$F-NMR: 300MHz, DMSO-d6, δ (ppm) -55 (s, 3F)}

Production Example 8

Synthesis of 4-phenylthio-1,8-naphthalic anhydride

[Intermediate (8)]

In dimethylacetamide (50 mL), potassium carbonate (2.1 g, 15 mmol) and thiophenol (1.8 g, 16 mmol) were dissolved to allow the contents to undergo a reaction for 1 hour at 70°C, after which 4-bromo-1,8-naphthalic anhydride (4.1 g, 15 mmol) was added thereto, and furthermore, the contents were stirred for 15 hours at 150°C. After being cooled to room temperature, the reaction liquid was charged into water (75 mL) and extracted with chloroform (75 mL), after which the chloroform layer was washed 2 times with aqueous 1 N hydrochloric acid (50 mL) and 3 times with water (50 mL) by liquid-liquid separating operation and transferred to a rotary evaporator, and the solvent was distilled off to obtain white solid matter. This white solid matter was washed with methanol to obtain the title compound [Intermediate (8)]. The product was identified by $^1$H-NMR. {$^1$H-NMR: 300MHz, DMSO-d6, $\delta$ (ppm) 8.7-8.5 (m, 3H), 7.9-7.6 (m, 4H), 7.4-7.0 (m, 5H)}

Production Example 9

Synthesis of 3-methoxy-1,8-naphthalic anhydride

[Intermediate (9)]

In acetone (50 mL), 3-hydroxy-1,8-naphthalic anhydride (3.2 g, 15 mmol) was dissolved, and to this solution, potassium carbonate (6.3 g, 45 mmol) was added and dimethyl sulfate (2.1 g, 16 mmol) was added dropwise over a period of 30 minutes with stirring at room temperature (25°C) to allow the contents to undergo a reaction for 3 hours at 65°C. The reaction liquid was charged into stirred 1 N hydrochloric acid (150 mL) contained in a beaker, and the precipitated white solid matter was filtered and washed to obtain the title compound [Intermediate (9)]. The product was identified by $^1$H-NMR. {$^1$H-NMR: 300MHz, DMSO-d6, $\delta$ (ppm)8.4-8.3(dd, 2H), 8.0(dd, 2H), 7.8 (t, 1H), 4.0(s, 3H)}

Production Example 10

Synthesis of 3-(2-ethylhexanoyloxy)-1,8-naphthalic anhydride [Intermediate (10)]

In acetonitrile (20 mL), 3-hydroxy-1,8-naphthalic anhydride (3.2 g, 15 mmol) and 2-ethylhexanoyl chloride (2.9 g, 18 mmol) were dissolved, and to this solution, pyridine (1.8 g, 22.5 mmol) was added dropwise over a period of 30 minutes with ice-bath cooling to allow the contents to undergo a reaction for 3 hours at room temperature (25°C). The reaction liquid was charged into stirred water (150 mL) contained in a beaker, and the precipitated white solid matter was filtered and washed to obtain the title compound [Intermediate (10)]. The product was identified by $^1$H-NMR. {$^1$H-NMR: 300MHz, DMSO-d6, $\delta$ (ppm) 8.6 (d, 1H), 8.3(m, 2H), 8.1(s, 1H), 7.8(t, 1H), 2.6(m, 1H), 1.9-1.6(m, 4H), 1.5-1.4(m, 4H), 1.1(t, 3H), 0.9(t, 3H)}

Production Example 11

Synthesis of 3-butyroyloxy-1,8-naphthalic anhydride

[Intermediate (11)]

In acetonitrile (20 mL), 3-hydroxy-1,8-naphthalic anhydride (3.2 g, 15 mmol) and butanoyl chloride (1.9 g, 18 mmol) were dissolved, and to this solution, pyridine (1.8 g, 22.5 mmol) was added dropwise over a period of 30 minutes with ice-bath cooling to allow the contents to undergo a reaction for 3 hours at room temperature (25°C). The reaction liquid was charged into stirred water (150 mL) contained in a beaker, and the precipitated white solid matter was filtered and washed to obtain the title compound [Intermediate (11)]. The product was identified by $^1$H-NMR. {$^1$H-NMR: 300MHz, DMSO-d6, $\delta$ (ppm) 8.6 (d, 1H), 8.3(m, 2H), 8.1(s, 1H), 7.8(t, 1H), 2.6(m, 2H), 1.9-1.6(m, 4H), 0.9(t, 3H)}

Production Example 12

Synthesis of 3-benzoyloxy-1,8-naphthalic anhydride

[Intermediate (12)]

In acetonitrile (20 mL), 3-hydroxy-1,8-naphthalic anhydride (3.2 g, 15 mmol) and benzoyl chloride (2.2 g, 18 mmol) were dissolved, and to this solution, pyridine (1.8 g, 22.5 mmol) was added dropwise over a period of 30 minutes with ice-bath cooling to allow the contents to undergo a reaction for 3 hours at room temperature (25°C). The reaction liquid was charged into stirred water (150 mL) contained in a beaker, and the precipitated white solid matter was filtered and washed to obtain the title compound [Intermediate (12)]. The product was identified by $^{1}$H-NMR. {$^{1}$H-NMR: 300MHz, DMSO-d6, δ(ppm)8.6(d, 1H), 8.3(m, 2H), 8.1(s, 1H), 8.0-8.9(m, 2H), 7.8(t, 1H), 7.6-7.3(m, 3H)}

Production Example 13

Synthesis of 3-pentafluorobenzoyloxy-1,8-naphthalic anhydride [Intermediate (13)]

In acetonitrile (20 mL), 3-hydroxy-1,8-naphthalic anhydride (3.2 g, 15 mmol) and pentafluorobenzoyl chloride (4.1 g, 18 mmol) were dissolved, and to this solution, pyridine (1.8 g, 22.5 mmol) was added dropwise over a period of 30 minutes with ice-bath cooling to allow the contents to undergo a reaction for 3 hours at room temperature (25°C). The reaction liquid was charged into stirred water (150 mL) contained in a beaker, and the precipitated white solid matter was filtered and washed to obtain the title compound [Intermediate (13)]. The product was identified by $^{1}$H-NMR and $^{19}$F-NMR. {$^{1}$H-NMR: 300MHz, DMSO-d6, δ (ppm) 8.6(d, 1H), 8.3(m, 2H), 8.1(s, 1H), 7.8(t, 1H), $^{19}$F-NMR: 300MHz, DMSO-d6, δ(ppm)-122(m, 2F), -131(m, 1F), -148 (m, 2F)}

Production Example 14

Synthesis of 3,6-di-tert-butyl-1,8-naphthalic anhydride

[Intermediate (14)]

In dichloromethane (50 mL), acenaphthene (2.3 g, 15 mmol) was dissolved, and to this solution, 2-chloro-2-methylpropane (3.5 g, 33 mmol) and aluminum chloride (0.04 g, 0.3 mmol) were added to allow the contents to be stirred for 1 hour at room temperature (25°C) and then allow the contents to undergo a reaction for 12 hours at 40°C. This reaction liquid was charged into methanol (500 mL) contained in a beaker, the solid matter was filtered and washed to obtain 2.3 g of white solid matter (3,6-di-tert-butylacenaphthene). In pyridine (10 mL), this white solid matter (1.0 g, 3.8 mmol) was dissolved, and to this solution, potassium permanganate (2.4 g, 15 mmol) dissolved in water (10 mL) and sodium hydroxide (0.4 g, 9 mmol) dissolved in water (10 mL) were added to allow the contents to undergo a reaction for 2 hours at 80°C. Solid matter was removed from the reaction liquid by filtration, hydrochloric acid was added to this filtrate until the liquid becomes acidic, and the precipitated white solid matter was filtered and washed to obtain the title compound [Intermediate (14)]. The product was identified by $^{1}$H-NMR. {$^{1}$H-NMR: 300MHz, DMSO-d6, δ (ppm) 7.7 (d, 2H), 7.4(d, 2H), 1.0(s, 9H)}

Production Example 15

Synthesis of 3,6-dineopentyl-1,8-naphthalic anhydride

[Intermediate (15)]

The title compound [Intermediate (15)] was obtained in the same manner as that in Production Example 14 except that the 2-chloro-2-methylpropane (3.5 g, 33 mmol) was changed to 2-chloro-2-methylbutane (3.5 g, 33 mmol). The product was identified by $^{1}$H-NMR. {$^{1}$H-NMR: 300MHz, DMSO-d6, δ(ppm)7.7(d, 2H), 7.4(d, 2H), 1.2(q, 2H), 1.0 (s, 6H), 0.9(t, 3H)}

Production Example 16

Synthesis of 3,6-diadamantyl-1,8-naphthalic anhydride

[Intermediate (16)]

The title compound [Intermediate (16)] was obtained in the same manner as that in Production Example 14 except that the 2-chloro-2-methylpropane (3.5 g, 33 mmol) was changed to 1-chloroadamantane (5.6 g, 33 mmol). The product was identified by $^1$H-NMR. {$^1$H-NMR: 300MHz, DMSO-d6, $\delta$(ppm)7.7(d, 2H), 7.4(d, 2H), 1.2-0.8(m, 15H)}

Production Example 17

Synthesis of 4-(nonafluoro-n-butyl)-1,8-naphthalic anhydride [Intermediate (17)]

In dimethyl sulfoxide (50 mL), 4-bromo-1,8-naphthalic anhydride (4.1 g, 15 mmol) and nonafluoro-n-butyl iodide (5.6 g, 16 mmol) were dissolved, and to this solution, copper powder (5.7 g) was added to allow the contents to be stirred for 2 hours at 140°C under a nitrogen atmosphere. After being cooled to room temperature, the reaction liquid was charged into water (75 mL) and extracted with chloroform (75 mL), after which the chloroform layer was washed 2 times with aqueous 1 N hydrochloric acid (50 mL) and 3 times with water (50 mL) by liquid-liquid separating operation and transferred to a rotary evaporator, and the solvent was distilled off to obtain white solid matter. This white solid matter was washed with methanol to obtain the title compound [Intermediate (17)]. The product was identified by $^1$H-NMR and $^{19}$F-NMR. {$^1$H-NMR: 300MHz, DMSO-d6, $\delta$ (ppm) 8.7-8.5 (m, 3H), 7.8(d, 1H), 7.7(t, 1H), $^{19}$F-NMR: 300MHz, DMSO-d6, $\delta$(ppm)-76(s, 3F), -100(m, 2F), -116(m, 2F), -121 (m, 2F)}

Production Example 18

Synthesis of 4-(heptadecafluoro-n-octyl)-1,8-naphthalic anhydride [Intermediate (18)]

The title compound [Intermediate (18)] was obtained in the same manner as that in Production Example 17 except that the nonafluoro-n-butyl iodide (5.6 g, 16 mmol) was changed to heptadecafluoro-n-octyl iodide (8.7 g, 16 mmol). The product was identified by $^1$H-NMR and $^{19}$F-NMR. {$^1$H-NMR: 300MHz, DMSO-d6, $\delta$ (ppm) 8.7-8.5 (m, 3H), 7.8(d, 1H), 7.7(t, 1H), $^{19}$F-NMR: 300MHz, DMSO-d6, $\delta$(ppm)-80(m, 3F), -100 (m, 2F), -116(m, 2F), -118 to -120(m, 8F), -124 (m, 2F)}

Production Example 19

Synthesis of 4-trimethylsilyl-1,8-naphthalic anhydride

[Intermediate (19)]

In a branched Schlenk tube equipped with a septum, 5-bromoacenaphthene (3.5 g, 15 mmol) and THF (50 mL) were placed, and to the contents in the vessel in an airtightly sealed state, a 1.6 mol/L hexane solution of n-butyllithium (10 mL) was slowly added via a syringe with acetone/dry ice-bath cooling and stirring, and furthermore, to the contents, trimethylsilyl chloride (2.4 g, 22.5 mmol) was added dropwise over a period of 15 minutes to allow the contents to undergo a reaction at room temperature (25°C) for 1 hour. This reaction liquid was charged into ice water (75 g) and extracted with chloroform (50 mL), after which the chloroform layer was separated by liquid-liquid separating operation, and furthermore, washed 2 times with water (50 mL) and 2 times with a liquid mixture (50 mL) of water/methanol = 1/1, and the solvent was removed on a rotary evaporator to obtain 4.0 g of white solid matter (4-trimethylsilylacenaphthene). In pyridine (10 mL), this white solid matter (0.9 g, 3.8 mmol) was dissolved, and to this solution, potassium permanganate (2.4 g, 15 mmol) dissolved in water (10 mL) and sodium hydroxide (0.4 g, 9 mmol) dissolved in water (10 mL) were added to allow the contents to undergo a reaction for 2 hours at 80°C. Solid matter was removed from the reaction liquid by filtration, hydrochloric acid was added to this filtrate until the liquid becomes acidic, and the precipitated white solid matter was filtered and washed to obtain the title compound [Intermediate (19)]. The product was identified by $^1$H-NMR. {$^1$H-NMR: 300MHz, Deuterated chloroform, $\delta$(ppm)8.5-8.4(m, 3H), 7.9(d, 1H), 7.7 (t, 1H), 0.4(s, 9H)}

Example 1

Synthesis of 4-isopropoxy-1,8-naphthalimide trifluoromethanesulfonate [Non-ionic photoacid generator (A-1)]

In pyridine (50 mL), 4-isopropoxy-1,8-naphthalic anhydride [Intermediate (2)] (4.1 g, 15 mmol) obtained in Production Example 2 was dissolved, and to this solution, hydroxyimide hydrochloride (1.5 g, 21 mmol) was added to allow the contents to undergo a reaction for 5 hours at 115°C, after which the reaction liquid was charged into a liquid mixture of hydrochloric acid (100 g) and water (100 mL) to collect a precipitate. In dichloromethane (30 mL), the obtained precipitate was dispersed, and to this dispersion, trifluoromethanesulfonic anhydride (7.4 g, 26 mmol) was added and pyridine (2.4 g, 30 mmol) was added dropwise over a period of 15 minutes with ice-bath cooling to allow the contents to undergo a reaction for 1 hour at room temperature (25°C) and furthermore allow the contents to undergo a reaction for 5 hours at 40°C. This reaction liquid was charged into aqueous 1 N hydrochloric acid (50 mL), the dichloromethane layer was separated by liquid-liquid separating operation, the dichloromethane layer was washed 2 times with aqueous 1 N hydrochloric acid (50 mL) and 2 times with water (50 mL), the solvent was removed on a rotary evaporator, and the obtained solid matter was recrystallized from isobutanol to obtain the title compound [Non-ionic photoacid generator (A-1)]. The product was identified by $^{1}$H-NMR and $^{19}$F-NMR. {$^{1}$H-NMR: 300MHz, Deuterated chloroform, $\delta$(ppm)8.6-8.4(m, 3H), 7.8(t, 1H), 7.3(d, 1H), 5.1(m, 1H), 1.4(d, 6H), $^{19}$F-NMR: 300MHz, Deuterated chloroform, $\delta$(ppm)-75(s, 3F)}

Example 2

Synthesis of 4-n-propoxy-1,8-naphthalimide

trifluoromethanesulfonate [Non-ionic photoacid generator (A-2)]

The title compound [Non-ionic photoacid generator (A-2)] was obtained in the same manner as that in Example 1 except that the 4-isopropoxy-1,8-naphthalic anhydride [Intermediate (2)] (4.1 g, 15 mmol) was changed to 4-n-propoxy-1,8-naphthalic anhydride [Intermediate (3)] (3.8 g, 15 mmol) obtained in Production Example 3. The product was identified by $^{1}$H-NMR and $^{19}$F-NMR. {$^{1}$H-NMR: 300MHz, Deuterated chloroform, $\delta$ (ppm) 8.6-8.4 (m, 3H), 7.8(t, 1H), 7.3(d, 1H), 3.7 (t, 2H), 1.6(m, 2H), 0.9(t, 3H), $^{19}$F-NMR:300MHz, Deuterated chloroform, $\delta$(ppm)-75(s, 3F)}

Example 3

Synthesis of 4-(2,2,3,3-tetrafluoro-1-propoxy)-1,8-naphthalimide trifluoromethanesulfonate [Non-ionic photoacid generator (A-3)]

The title compound [Non-ionic photoacid generator (A-3)] was obtained in the same manner as that in Example 1 except that the 4-isopropoxy-1,8-naphthalic anhydride [Intermediate (2)] (4.1 g, 15 mmol) was changed to 4-(2,2,3,3-tetrafluoro-1-propoxy)-1,8-naphthalic anhydride [Intermediate (4)] (5.1 g, 15 mmol) obtained in Production Example 4. The product was identified by $^{1}$H-NMR and $^{19}$F-NMR. {$^{1}$H-NMR: 300MHz, Deuterated chloroform, $\delta$(ppm)8.6-8.4(m, 3H), 7.8(t, 1H), 7.3(d, 1H), 5.9(tt, 1H), 3.8(t, 2H), $^{19}$F-NMR:300MHz, Deuterated chloroform, $\delta$(ppm)-66(s, 3F), -122(d, 2F), -133 (d, 2F)}

Example 4

Synthesis of 4-phenylacetyl-1,8-naphthalimide trifluoromethanesulfonate [Non-ionic photoacid generator (A-4)]

The title compound [Non-ionic photoacid generator (A-4)] was obtained in the same manner as that in Example 1 except that the 4-isopropoxy-1,8-naphthalic anhydride [Intermediate (2)] (4.1 g, 15 mmol) was changed to 4-phenylacetyl-1,8-naphthalic anhydride [Intermediate (5)] (4.7 g, 15 mmol) obtained in Production Example 5. The product was identified by $^{1}$H-NMR and $^{19}$F-NMR. {$^{1}$H-NMR: 300MHz, Deuterated chloroform, $\delta$ (ppm) 8.6-8.4 (m, 5H), 7.9-7.7(m, 3H), 7.3(d, 1H), 7.1(t, 1H), $^{19}$F-NMR: 300MHz, Deuterated chloroform, $\delta$ (ppm) -75 (s, 3F)}

Example 5

Synthesis of 4-pentafluorophenoxy-1,8-naphthalimide trifluoromethanesulfonate [Non-ionic photoacid generator (A-5)]

The title compound [Non-ionic photoacid generator (A-5)] was obtained in the same manner as that in Example 1 except that the 4-isopropoxy-1,8-naphthalic anhydride [Intermediate (2)] (4.1 g, 15 mmol) was changed to 4-pentafluorophenoxy-1,8-naphthalic anhydride [Intermediate (6)] (5.9 g, 15 mmol) obtained in Production Example 6. The product was identified by $^{1}$H-NMR and $^{19}$F-NMR. {$^{1}$H-NMR: 300MHz, Deuterated chloroform, δ (ppm) 8.6-8.4 (m, 3H), 7.9(t, 1H), 6.9(d, 1H), $^{19}$F-NMR: 300MHz, Deuterated chloroform, δ(ppm)-146(d, 2F), -149(t, 1F), -155(t, 2F)}

Example 6

Synthesis of 4-(3-trifluoromethylphenoxy)-1,8-naphthalimide trifluoromethanesulfonate [Non-ionic photoacid generator (A-6)]

The title compound [Non-ionic photoacid generator (A-6)] was obtained in the same manner as that in Example 1 except that the 4-isopropoxy-1,8-naphthalic anhydride [Intermediate (2)] (4.1 g, 15 mmol) was changed to 4-(3-trifluoromethylphenoxy)-1,8-naphthalic anhydride [Intermediate (7)] (5.5 g, 15 mmol) obtained in Production Example 7. The product was identified by $^{1}$H-NMR and $^{19}$F-NMR. {$^{1}$H-NMR: 300MHz, Deuterated chloroform, δ (ppm) 8.7-8.5 (m, 3H), 7.9-7.6(m, 4H), 7.1-7.0(m, 2H), $^{19}$F-NMR: 300MHz, Deuterated chloroform, δ(ppm)-55(s, 3F), -66(s, 3F)}

Example 7

Synthesis of 4-phenylthio-1,8-naphthalimide trifluoromethanesulfonate [Non-ionic photoacid generator (A-7)]

The title compound [Non-ionic photoacid generator (A-7)] was obtained in the same manner as that in Example 1 except that the 4-isopropoxy-1,8-naphthalic anhydride [Intermediate (2)] (4.1 g, 15 mmol) was changed to 4-phenylthio-1,8-naphthalic anhydride [Intermediate (8)] (4.8 g, 15 mmol) obtained in Production Example 8. The product was identified by $^{1}$H-NMR and $^{19}$F-NMR. {$^{1}$H-NMR: 300MHz, Deuterated chloroform, δ(ppm)8.7-8.5(m, 3H), 7.9-7.6(m, 4H), 7.4-7.0(m, 5H), $^{19}$F-NMR: 300MHz, Deuterated chloroform, δ (ppm) -75 (s, 3F)}

Example 8

Synthesis of 3-methoxy-1,8-naphthalimide

trifluoromethanesulfonate [Non-ionic photoacid generator (A-8)]

The title compound [Non-ionic photoacid generator (A-8)] was obtained in the same manner as that in Example 1 except that the 4-isopropoxy-1,8-naphthalic anhydride [Intermediate (2)] (4.1 g, 15 mmol) was changed to 3-methoxy-1,8-naphthalic anhydride [Intermediate (9)] (3.6 g, 15 mmol) obtained in Production Example 9. The product was identified by $^{1}$H-NMR and $^{19}$F-NMR. {$^{1}$H-NMR: 300MHz, Deuterated chloroform, δ(ppm)8.4-8.3(dd, 2H), 8.0(dd, 2H), 7.8(t, 1H), 4.0(s, 3H)$^{19}$F-NMR: 300MHz, Deuterated chloroform, δ(ppm)-75(s, 3F)}

Example 9

Synthesis of 3-(2-ethylhexanoyloxy)-1,8-naphthalimide trifluoromethanesulfonate [Non-ionic photoacid generator (A-9)]

The title compound [Non-ionic photoacid generator (A-9)] was obtained in the same manner as that in Example 1 except that the 4-isopropoxy-1,8-naphthalic anhydride [Intermediate (2)] (4.1 g, 15 mmol) was changed to 3-(2-ethylhexanoyloxy)-1,8-naphthalic anhydride [Intermediate (10)] (5.3 g, 15 mmol) obtained in Production Example 10. The product was identified by $^{1}$H-NMR and $^{19}$F-NMR. {$^{1}$H-NMR: 300MHz, Deuterated chloroform, δ(ppm)8.6(d, 1H), 8.3(m, 2H), 8.1(s, 1H), 7.8(t, 1H), 2.6(m, 1H), 1.9-1.6(m, 4H), 1.5-1.4(m, 4H), 1.1(t, 3H), 0.9(t, 3H), $^{19}$F-NMR: 300MHz, Deuterated chloroform, δ(ppm)-75(s, 3F)}

Example 10

Synthesis of 3-butyroyloxy-1,8-naphthalimide trifluoromethanesulfonate [Non-ionic photoacid generator (A-10)]

The title compound [Non-ionic photoacid generator (A-10)] was obtained in the same manner as that in Example 1 except that the 4-isopropoxy-1,8-naphthalic anhydride [Intermediate (2)] (4.1 g, 15 mmol) was changed to 3-butyroyloxy-1,8-naphthalic anhydride [Intermediate (11)] (4.5 g, 15 mmol) obtained in Production Example 11. The product was identified by $^{1}$H-NMR and $^{19}$F-NMR. {$^{1}$H-NMR: 300MHz, Deuterated chloroform, δ(ppm)8.6(d, 1H), 8.3(m, 2H), 8.1(s, 1H), 7.8(t, 1H), 2.6(m, 2H), 1.9-1.6(m, 4H), 0.9(t, 3H)$^{19}$F-NMR: 300MHz, Deuterated chloroform, δ(ppm)-75(s, 3F)}

Example 11

Synthesis of 3-benzoyloxy-1,8-naphthalimide trifluoromethanesulfonate [Non-ionic photoacid generator (A-11)]

The title compound [Non-ionic photoacid generator (A-11)] was obtained in the same manner as that in Example 1 except that the 4-isopropoxy-1,8-naphthalic anhydride [Intermediate (2)] (4.1 g, 15 mmol) was changed to 3-benzoyloxy-1,8-naphthalic anhydride [Intermediate (12)] (5.0 g, 15 mmol) obtained in Production Example 12. The product was identified by $^1$H-NMR and $^{19}$F-NMR. {$^1$H-NMR: 300MHz, Deuterated chloroform, δ(ppm)8.6(d, 1H), 8.3(m, 2H), 8.1(s, 1H), 8.0-8.9(m, 2H), 7.8(t, 1H), 7.6-7.3(m, 3H), $^{19}$F-NMR: 300MHz, Deuterated chloroform, δ(ppm)-75(s, 3F)}

Example 12

Synthesis of 3-pentafluorobenzoyloxy-1,8-naphthalimide trifluoromethanesulfonate [Non-ionic photoacid generator (A-12)]

The title compound [Non-ionic photoacid generator (A-12)] was obtained in the same manner as that in Example 1 except that the 4-isopropoxy-1,8-naphthalic anhydride [Intermediate (2)] (4.1 g, 15 mmol) was changed to 3-pentafluorobenzoyloxy-1,8-naphthalic anhydride [Intermediate (13)] (3.6 g, 15 mmol) obtained in Production Example 13. The product was identified by $^1$H-NMR and $^{19}$F-NMR. {$^1$H-NMR: 300MHz, Deuterated chloroform, δ(ppm)8.6(d, 1H), 8.3(m, 2H), 8.1(s, 1H), 7.8(t, 1H), $^{19}$F-NMR: 300MHz, Deuterated chloroform, δ(ppm)-75(s, 3F), -122(m, 2F), - 131 (m, 1F), -148 (m, 2F)}

Example 13

Synthesis of 3,6-di-tert-butyl-1,8-naphthalimide trifluoromethanesulfonate [Non-ionic photoacid generator (A-13)]

The title compound [Non-ionic photoacid generator (A-13)] was obtained in the same manner as that in Example 1 except that the 4-isopropoxy-1,8-naphthalic anhydride [Intermediate (2)] (4.1 g, 15 mmol) was changed to 3,6-di-tert-butyl-1,8-naphthalic anhydride [Intermediate (14)] (4.9 g, 15 mmol) obtained in Production Example 14. The product was identified by $^1$H-NMR and $^{19}$F-NMR. {$^1$H-NMR: 300MHz, Deuterated chloroform, δ (ppm)7.7(d, 2H), 7.4(d, 2H), 1.0 (s, 9H), $^{19}$F-NMR: 300MHz, Deuterated chloroform, δ(ppm)-75(s, 3F)}

Example 14

Synthesis of 3,6-dineopentyl-1,8-naphthalimide trifluoromethanesulfonate [Non-ionic photoacid generator (A-14)]

The title compound [Non-ionic photoacid generator (A-14)] was obtained in the same manner as that in Example 1 except that the 4-isopropoxy-1,8-naphthalic anhydride [Intermediate (2)] (4.1 g, 15 mmol) was changed to 3,6-dineopentyl-1,8-naphthalic anhydride [Intermediate (15)] (5.3 g, 15 mmol) obtained in Production Example 15. The product was identified by $^1$H-NMR and $^{19}$F-NMR. {$^1$H-NMR: 300MHz, Deuterated chloroform, δ(ppm)7.7(d, 2H), 7.4(d, 2H), 1.2(q, 2H), 1.0(s, 6H), 0.9(t, 3H), $^{19}$F-NMR: 300MHz, Deuterated chloroform, δ(ppm)-75(s, 3F)}

Example 15

Synthesis of 3,6-diadamantyl-1,8-naphthalimide trifluoromethanesulfonate [Non-ionic photoacid generator (A-15)]

The title compound [Non-ionic photoacid generator (A-15)] was obtained in the same manner as that in Example 1 except that the 4-isopropoxy-1,8-naphthalic anhydride [Intermediate (2)] (4.1 g, 15 mmol) was changed to 3,6-diadamantyl-1,8-naphthalic anhydride [Intermediate (16)] (7.2 g, 15 mmol) obtained in Production Example 16. The product was identified by $^1$H-NMR and $^{19}$F-NMR. {$^1$H-NMR: 300MHz, Deuterated chloroform, δ(ppm)7.7(d, 2H), 7.4(d, 2H), 1.2-0.8(m, 15H), $^{19}$F-NMR: 300MHz, Deuterated chloroform, δ(ppm)-75(s, 3F)}

Example 16

Synthesis of 4-(nonafluorobutyl)-1,8-naphthalimide trifluoromethanesulfonate [Non-ionic photoacid generator (A-16)]

The title compound [Non-ionic photoacid generator (A-16)] was obtained in the same manner as that in Example 1 except that the 4-isopropoxy-1,8-naphthalic anhydride [Intermediate (2)] (4.1 g, 15 mmol) was changed to 4-(nonafluorobutyl)-1,8-naphthalic anhydride [Intermediate (17)] (6.5 g, 15 mmol) obtained in Production Example 17. The product was identified by $^1$H-NMR and $^{19}$F-NMR. {$^1$H-NMR: 300MHz, Deuterated chloroform, δ(ppm)8.8(m, 2H), 8.7(d, 1H), 8.2(d, 1H), 7.9(t, 1H), $^{19}$F-NMR: 300MHz, Deuterated chloroform, δ(ppm)-66(s, 3F), -76(s, 3F), -100(m, 2F), - 116 (m, 2F), -121 (m, 2F)}

Example 17

Synthesis of 4-(heptadecafluoro-n-octyl)-1,8-naphthalimide trifluoromethanesulfonate [Non-ionic photoacid generator (A-17)]

The title compound [Non-ionic photoacid generator (A-17)] was obtained in the same manner as that in Example 1 except that the 4-isopropoxy-1,8-naphthalic anhydride [Intermediate (2)] (4.1 g, 15 mmol) was changed to 4-(heptadecafluoro-n-octyl)-1,8-naphthalic anhydride [Intermediate (18)] (8.7 g, 15 mmol) obtained in Production Example 18. The product was identified by $^1$H-NMR and $^{19}$F-NMR. {$^1$H-NMR: 300MHz, Deuterated chloroform, δ(ppm)8.8(m, 2H), 8.7(d, 1H), 8.2(d, 1H), 7.9(t, 1H), $^{19}$F-NMR: 300MHz, Deuterated chloroform, δ(ppm)-66(s, 3F), -76(s, 3F), -100(m, 2F), -116 (m, 2F), -121 (m, 2F)}

Example 18

Synthesis of 4-trimethylsilyl-1,8-naphthalimide trifluoromethanesulfonate [Non-ionic photoacid generator (A-18)]

The title compound [Non-ionic photoacid generator (A-18)] was obtained in the same manner as that in Example 1 except that the 4-isopropoxy-1,8-naphthalic anhydride [Intermediate (2)] (4.1 g, 15 mmol) was changed to 4-trimethylsilyl-1,8-naphthalic anhydride [Intermediate (19)] (4.5 g, 15 mmol) obtained in Production Example 19. The product was identified by $^1$H-NMR and $^{19}$F-NMR. {$^1$H-NMR: 300MHz, Deuterated chloroform, δ(ppm)8.5-8.4(m, 3H), 7.9(d, 1H), 7.7(t, 1H), 0.4(s, 9H), $^{19}$F-NMR: 300MHz, Deuterated chloroform, δ(ppm)-75(s, 3F)}

Example 19

Synthesis of 4-chloro-1,8-naphthalimide

trifluoromethanesulfonate [Non-ionic photoacid generator (A-19)]

The title compound [Non-ionic photoacid generator (A-19)] was obtained in the same manner as that in Example 1 except that the 4-isopropoxy-1,8-naphthalic anhydride [Intermediate (2)] (4.1 g, 15 mmol) was changed to 4-chloro-1,8-naphthalic anhydride (3.7 g, 15 mmol). The product was identified by $^1$H-NMR and $^{19}$F-NMR. {$^1$H-NMR: 300MHz, Deuterated chloroform, δ(ppm)8.5-8.4(m, 3H), 7.9(d, 1H), 7.7(t, 1H), $^{19}$F-NMR: 300MHz, Deuterated chloroform, δ(ppm)-75(s, 3F)}

Example 20

Synthesis of 4-bromo-1,8-naphthalimide

trifluoromethanesulfonate [Non-ionic photoacid generator (A-20)]

The title compound [Non-ionic photoacid generator (A-20)] was obtained in the same manner as that in Example 1 except that the 4-isopropoxy-1,8-naphthalic anhydride [Intermediate (2)] (4.1 g, 15 mmol) was changed to 4-bromo-1,8-naphthalic anhydride (4.4 g, 15 mmol). The product was identified by $^1$H-NMR and $^{19}$F-NMR. {$^1$H-NMR: 300MHz, Deuterated chloroform, δ(ppm)8.5-8.4(m, 3H), 7.9(d, 1H), 7.7(t, 1H), $^{19}$F-NMR: 300MHz, Deuterated chloroform, δ(ppm)-75 (s, 3F)}

Comparative Example 1

Synthesis of 1,8-naphthalimide trifluoromethanesulfonate

[Non-ionic photoacid generator (A'-1)]

[Chemical 3]

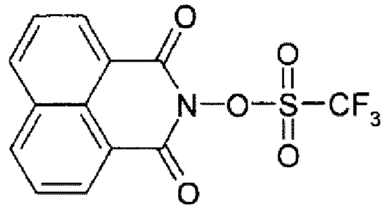

A non-ionic photoacid generator (A'-1) (0.4 g, 1.0 mmol) was obtained in the same manner as that in Example 1 except that the 4-isopropoxy-1,8-naphthalic anhydride [Intermediate (2)] (4.1 g, 15 mmol) was changed to naphthalic anhydride (3.2 g, 15 mmol).

Comparative Example 2

<Synthesis of Ionic photoacid generator (A'-2)>

[Chemical 4]

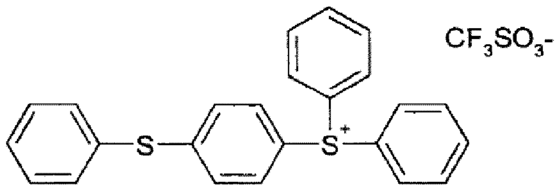

To a stirred mixture of diphenyl sulfoxide (12.1 g), diphenyl sulfide (9.3 g) and methanesulfonic acid (43.0 g), acetic anhydride (7.9 g) was added dropwise to allow the contents to undergo a reaction for 5 hours at 40 to 50°C, after which the contents were cooled to 25°C. This reaction solution was charged into an aqueous potassium trifluoromethanesulfonate solution (121 g) and the contents were stirred for 8 hours at 50°C, whereupon a slightly viscous yellow oily substance was precipitated. This oily substance was extracted with ethyl acetate, the organic layer was washed several times with water, and then, the solvent was distilled off from the organic layer. The obtained residue was added with toluene to be dissolved therein, after which hexane was added to the solution, and the contents were thoroughly stirred for 1 hour at 10°C, and then, allowed to settle. After 1 hour, since the solution separated into two layers, the upper layer was removed by liquid-liquid separation. The residual lower layer was added with hexane and the contents were thoroughly mixed at 25°C, whereupon pale yellow crystals were precipitated. The crystals were filtered and dried under reduced pressure to obtain an ionic photoacid generator (A'-2).

Example 21

Synthesis of 4-isopropoxy-1,8-naphthalimide pentafluorobenzenesulfonate [Non-ionic photoacid generator (A-21)]

The title compound [Non-ionic photoacid generator (A-21)] was obtained in the same manner as that in Example 1 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.6-8.4(m, 3H), 7.8(t, 1H), 7.3(d, 1H), 5.1(m, 1H), 1.4(d, 6H), $^{19}$F-NMR: 300MHz, Deuterated chloroform, $\delta$(ppm)-125(m, 2F), - 133 (m, 1F), -151 (m, 2F)}

Example 22

Synthesis of 4-n-propoxy-1,8-naphthalimide

pentafluorobenzenesulfonate [Non-ionic photoacid generator (A-22)]

The title compound [Non-ionic photoacid generator (A-22)] was obtained in the same manner as that in Example 2 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.6-8.4(m, 3H), 7.8 (t, 1H), 7.3(d, 1H), 3.7(t, 2H), 1.6(m, 2H), 0.9(t, 3H), $^{19}$F-NMR: 300MHz, Deuterated chloroform, $\delta$(ppm)-125 (m, 2F), -133 (m, 1F), -151 (m, 2F)}

Example 23

Synthesis of 4-(2,2,3,3-tetrafluoro-1-propoxy)-1,8-naphthalimide pentafluorobenzenesulfonate [Non-ionic photoacid generator (A-23)]

The title compound [Non-ionic photoacid generator (A-23)] was obtained in the same manner as that in Example 3 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.6-8.4(m, 3H), 7.8(t, 1H), 7.3(d, 1H), 5.9(tt, 1H), 3.8(t, 2H), $^{19}$F-NMR: 300MHz, Deuterated chloroform, $\delta$(ppm)-122(d, 2F), - 125 (m, 2F), -133 (d, 2F), -133 (m, 1F), -151 (m, 2F)}

Example 24

Synthesis of 4-phenylacetyl-1,8-naphthalimide pentafluorobenzenesulfonate [Non-ionic photoacid generator (A-24)]

The title compound [Non-ionic photoacid generator (A-24)] was obtained in the same manner as that in Example 4 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.6-8.4(m, 5H), 7.9-7.7(m, 3H), 7.3(d, 1H), 7.1(t, 1H), $^{19}$F-NMR:300MHz, Deuterated chloroform, $\delta$(ppm)-125(m, 2F), - 133 (m, 1F), -151 (m, 2F)}

Example 25

Synthesis of 4-pentafluorophenoxy-1,8-naphthalimide pentafluorobenzenesulfonate [Non-ionic photoacid generator (A-25)]

The title compound [Non-ionic photoacid generator (A-25)] was obtained in the same manner as that in Example 5 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.6-8.4(m, 3H), 7.8(d, 1H), 7.1(t, 1H), $^{19}$F-NMR: 300MHz, Deuterated chloroform, $\delta$(ppm)-125(m, 2F), -133(m, 1F),-146 (d, 2F), -149 (t, 1F), -151 (m, 2F), 155 (t, 2F)}

Example 26

Synthesis of 4-(3-trifluoromethylphenoxy)-1,8-naphthalimide pentafluorobenzenesulfonate [Non-ionic photoacid generator (A-26)]

The title compound [Non-ionic photoacid generator (A-26)] was obtained in the same manner as that in Example 6 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.7-8.5(m, 3H), 7.9-7.6(m, 4H), 7.1-7.0(m, 2H), $^{19}$F-NMR: 300MHz, Deuterated chloroform, $\delta$(ppm)-55(s, 1F), -75(s, 3F), -125(m, 2F), -133 (m, 1F), -151 (m, 2F)}

Example 27

Synthesis of 4-phenylthio-1,8-naphthalimide pentafluorobenzenesulfonate [Non-ionic photoacid generator (A-27)]

The title compound [Non-ionic photoacid generator (A-27)] was obtained in the same manner as that in Example 7 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, δ(ppm)8.7-8.5(m, 3H), 7.9-7.6(m, 4H), 7.4-7.0(m, 5H), $^{19}$F-NMR: 300MHz, Deuterated chloroform, δ(ppm)-125(m, 2F), -133(m, 1F),-151 (m, 2F)}

Example 28

Synthesis of 3-methoxy-1,8-naphthalimide

pentafluorobenzenesulfonate [Non-ionic photoacid generator (A-28)]

The title compound [Non-ionic photoacid generator (A-28)] was obtained in the same manner as that in Example 8 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, δ(ppm)8.4-8.3(dd, 2H), 8.0(dd, 2H), 7.8(t, 1H), 4.0 (s, 3H) $^{19}$F-NMR:300MHz, Deuterated chloroform, δ(ppm), -125(m, 2F), - 133 (m, 1F), -151 (m, 2F)}

Example 29

Synthesis of 3-(2-ethylhexanoyloxy)-1,8-naphthalimide pentafluorobenzenesulfonate [Non-ionic photoacid generator (A-29)]

The title compound [Non-ionic photoacid generator (A-29)] was obtained in the same manner as that in Example 9 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, δ(ppm)8.6(d, 1H), 8.3(m, 2H), 8.1(s, 1H), 7.8(t, 1H), 2.6(m, 1H), 1.9-1.6(m, 4H), 1.5-1.4(m, 4H), 1.1(t, 3H), 0.9(t, 3H), $^{19}$F-NMR: 300MHz, Deuterated chloroform, δ(ppm), -125(m, 2F), -133 (m, 1F), -151 (m, 2F)}

Example 30

Synthesis of 3-butyroyloxy-1,8-naphthalimide pentafluorobenzenesulfonate [Non-ionic photoacid generator (A-30)]

The title compound [Non-ionic photoacid generator (A-30)] was obtained in the same manner as that in Example 10 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, δ(ppm)8.6(d, 1H), 8.3(m, 2H), 8.1(s, 1H), 7.8(t, 1H), 2.6(m, 2H), 1.9-1.6(m, 4H), 0.9(t, 3H), $^{19}$F-NMR: 300MHz, Deuterated chloroform, δ(ppm), -125(m, 2F), -133(m, 1F), -151(m, 2F)}

Example 31

Synthesis of 3-benzoyloxy-1,8-naphthalimide

pentafluorobenzenesulfonate [Non-ionic photoacid generator (A-31)]

The title compound [Non-ionic photoacid generator (A-31)] was obtained in the same manner as that in Example 11 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, δ(ppm)8.6(d, 1H), 8.3(m, 2H), 8.1(s, 1H), 8.0-8.9(m, 2H), 7.8(t, 1H), 7.6-7.3(m, 3H), $^{19}$F-NMR: 300MHz, Deuterated chloroform, δ(ppm), -125(m, 2F), -133(m, 1F), -151(m, 2F)}

Example 32

Synthesis of 3-pentafluorobenzoyloxy-1,8-naphthalimide pentafluorobenzenesulfonate [Non-ionic photoacid generator (A-32)]

The title compound [Non-ionic photoacid generator (A-32)] was obtained in the same manner as that in Example 12 except that the trifluoromethanesulfonic anhydride (7.4 g, 26 mmol) was changed to pentafluorobenzenesulfonyl chloride (6.9 g, 26 mmol). The product was identified by $^{1}$H-NMR and $^{19}$F-NMR. {$^{1}$H-NMR: 300MHz, Deuterated chloroform, $\delta$(ppm)8.6(d, 1H), 8.3(m, 2H), 8.1(s, 1H), 7.8(t, 1H), $^{19}$F-NMR:300MHz, Deuterated chloroform, $\delta$(ppm)-122(m, 2F), - 123 (m, 2F), -131 (m, 1F), -133 (m, 1F), -148 (m, 2F), -151 (m, 2F)}

Example 33

Synthesis of 3,6-di-tert-butyl-1,8-naphthalimide pentafluorobenzenesulfonate [Non-ionic photoacid generator (A-33)]

The title compound [Non-ionic photoacid generator (A-33)] was obtained in the same manner as that in Example 13 except that the trifluoromethanesulfonic anhydride (7.4 g, 26 mmol) was changed to pentafluorobenzenesulfonyl chloride (6.9 g, 26 mmol). The product was identified by $^{1}$H-NMR and $^{19}$F-NMR. {$^{1}$H-NMR: 300MHz, Deuterated chloroform, $\delta$(ppm)7.7(d, 2H), 7.4 (d, 2H), 1.0(s, 9H), $^{19}$F-NMR: 300MHz, Deuterated chloroform, $\delta$(ppm), -125(m, 2F), -133(m, 1F), - 151 (m, 2F)}

Example 34

Synthesis of 3,6-dineopentyl-1,8-naphthalimide pentafluorobenzenesulfonate [Non-ionic photoacid generator (A-34)]

The title compound [Non-ionic photoacid generator (A-34)] was obtained in the same manner as that in Example 14 except that the trifluoromethanesulfonic anhydride (7.4 g, 26 mmol) was changed to pentafluorobenzenesulfonyl chloride (6.9 g, 26 mmol). The product was identified by $^{1}$H-NMR and $^{19}$F-NMR. {$^{1}$H-NMR: 300MHz, Deuterated chloroform, $\delta$(ppm)7.7(d, 2H), 7.4(d, 2H), 1.2(q, 2H), 1.0 (s, 6H), 0.9(t, 3H), $^{19}$F-NMR: 300MHz, Deuterated chloroform, $\delta$(ppm)-125(m, 2F), -133 (m, 1F), -151 (m, 2F)}

Example 35

Synthesis of 3,6-diadamantyl-1,8-naphthalimide pentafluorobenzenesulfonate [Non-ionic photoacid generator (A-35)]

The title compound [Non-ionic photoacid generator (A-35)] was obtained in the same manner as that in Example 15 except that the trifluoromethanesulfonic anhydride (7.4 g, 26 mmol) was changed to pentafluorobenzenesulfonyl chloride (6.9 g, 26 mmol). The product was identified by $^{1}$H-NMR and $^{19}$F-NMR. {$^{1}$H-NMR: 300MHz, Deuterated chloroform, $\delta$(ppm)7.7(d, 2H), 7.4(d, 2H), 1.2-0.8 (m, 15H), $^{19}$F-NMR: 300MHz, Deuterated chloroform, $\delta$(ppm)-125(m, 2F), -133(m, 1F), -151 (m, 2F)}

Example 36

Synthesis of 4-(nonafluorobutyl)-1,8-naphthalimide pentafluorobenzenesulfonate [Non-ionic photoacid generator (A-36)]

The title compound [Non-ionic photoacid generator (A-36)] was obtained in the same manner as that in Example 16 except that the trifluoromethanesulfonic anhydride (7.4 g, 26 mmol) was changed to pentafluorobenzenesulfonyl chloride (6.9 g, 26 mmol). The product was identified by $^{1}$H-NMR and $^{19}$F-NMR. {$^{1}$H-NMR: 300MHz, Deuterated chloroform, $\delta$(ppm)8.8(m, 2H), 8.7(d, 1H), 8.2(d, 1H), 7.9(t, 1H), $^{19}$F-NMR:300MHz, Deuterated chloroform, $\delta$(ppm)-76(s, 3F), -100(m, 2F), -116 (m, 2F), -121 (m, 2F), -125 (m, 2F), -133 (m, 1F),-151 (m, 2F)}

Example 37

Synthesis of 4-(heptadecafluoro-n-octyl)-1,8-naphthalimide pentafluorobenzenesulfonate [Non-ionic photoacid generator (A-37)]

The title compound [Non-ionic photoacid generator (A-37)] was obtained in the same manner as that in Example 17 except that the trifluoromethanesulfonic anhydride (7.4 g, 26 mmol) was changed to pentafluorobenzenesulfonyl

chloride (6.9 g, 26 mmol). The product was identified by $^1$H-NMR and $^{19}$F-NMR. {$^1$H-NMR: 300MHz, Deuterated chloroform, $\delta$(ppm)8.8(m, 2H), 8.7(d, 1H), 8.2(d, 1H), 7.9(t, 1H), $^{19}$F-NMR:300MHz, Deuterated chloroform, $\delta$(ppm)-80(m, 3F), -100(m, 2F), -116(m, 2F), -118 to -120(m, 8F), -124 (m, 2F), -125 (m, 2F), -133 (m, 1F), -151 (m, 2F)}

Example 38

Synthesis of 4-trimethylsilyl-1,8-naphthalimide pentafluorobenzenesulfonate [Non-ionic photoacid generator (A-38)]

The title compound [Non-ionic photoacid generator (A-38)] was obtained in the same manner as that in Example 18 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.5-8.4(m, 3H), 7.9(d, 1H), 7.7(t, 1H), 0.4(s, 9H), $^{19}$F-NMR:300MHz, Deuterated chloroform, $\delta$(ppm)-125(m, 2F), - 133 (m, 1F), -151 (m, 2F)}

Example 39

Synthesis of 4-chloro-1,8-naphthalimide

pentafluorobenzenesulfonate [Non-ionic photoacid generator (A-39)]

The title compound [Non-ionic photoacid generator (A-39)] was obtained in the same manner as that in Example 19 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.5-8.4(m, 3H), 7.9(d, 1H), 7.7(t, 1H), $^{19}$F-NMR:300MHz, Deuterated chloroform, $\delta$(ppm)-125(m, 2F), -133(m, 1F),-151 (m, 2F)}

Example 40

Synthesis of 4-bromo-1,8-naphthalimide

pentafluorobenzenesulfonate [Non-ionic photoacid generator (A-40)]

The title compound [Non-ionic photoacid generator (A-40)] was obtained in the same manner as that in Example 20 except that the trifluoromethanesulfonic anhydride (7.4 g, 26 mmol) was changed to pentafluorobenzenesulfonyl chloride (6.9 g, 26 mmol). The product was identified by $^1$H-NMR and $^{19}$F-NMR. {$^1$H-NMR: 300MHz, Deuterated chloroform, $\delta$(ppm)8.5-8.4(m, 3H), 7.9(d, 1H), 7.7(t, 1H), $^{19}$F-NMR:300MHz, Deuterated chloroform, $\delta$(ppm) -125 (m, 2F), -133(m, 1F),-151 (m, 2F)}

Comparative Example 3

Synthesis of 1,8-naphthalimide pentafluorobenzenesulfonate

[Non-ionic photoacid generator (A'-3)]

The title compound [Non-ionic photoacid generator (A'-3)] was obtained in the same manner as that in Comparative Example 1 except that the trifluoromethanesulfonic anhydride (7.4 g, 26 mmol) was changed to pentafluorobenzenesulfonyl chloride (6.9 g, 26 mmol).

Comparative Example 4

<Synthesis of Ionic photoacid generator (A'-4)>

An ionic photoacid generator (A'-4) was obtained in the same manner as that in Comparative Example 2 except that the aqueous potassium trifluoromethanesulfonate solution was changed to an aqueous potassium pentafluorobenzenesulfonate solution.

Example 41

Synthesis of 4-isopropoxy-1,8-naphthalimide-(+)-10-camphorsulfonate [Non-ionic photoacid generator (A-41)]

The title compound [Non-ionic photoacid generator (A-41)] was obtained in the same manner as that in Example 1 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, $\delta$(ppm)8.6-8.4(m, 3H), 7.8(t, 1H), 7.3(d, 1H), 5.1(m, 1H), 3.0(d, 1H), 2.5(m, 2H), 2.3(m, 1H), 1.9-1.7(m, 3H), 1.4(d, 6H), 1.4-1.2(m, 2H), 1.0(s, 3H), 0.8(s, 3H)}

Example 42

Synthesis of 4-n-propoxy-1,8-naphthalimide-(+)-10-camphorsulfonate [Non-ionic photoacid generator (A-42)]

The title compound [Non-ionic photoacid generator (A-42)] was obtained in the same manner as that in Example 2 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, $\delta$(ppm)8.6-8.4(m, 3H), 7.8(t, 1H), 7.3(d, 1H), 3.7(t, 2H), 3.0(d, 1H), 2.5(m, 2H), 2.3(m, 1H), 1.9-1.7(m, 3H), 1.6(m, 2H), 1.4-1.2(m, 2H), 1.0(s, 3H), 0.9(t, 3H), 0.8(s, 3H)}

Example 43

Synthesis of 4-(2,2,3,3-tetrafluoro-1-propoxy)-1,8-naphthalimide-(+)-10-camphorsulfonate [Non-ionic photoacid generator (A-43)]

The title compound [Non-ionic photoacid generator (A-43)] was obtained in the same manner as that in Example 3 except that the trifluoromethanesulfonic anhydride (7.4 g, 26 mmol) was changed to (+)-10-camphorsulfonyl chloride (6.5 g, 26 mmol). The product was identified by $^1$H-NMR and $^{19}$ F-NMR. {$^1$H-NMR: 300MHz, Deuterated chloroform, $\delta$(ppm)8.6-8.4(m, 3H), 7.8(t, 1H), 7.3(d, 1H), 5.9(tt, 1H), 3.8(t, 2H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, $\delta$(ppm) -122 (d, 2F), -133(d, 2F)}

Example 44

Synthesis of 4-phenylacetyl-1,8-naphthalimide-(+)-10-camphorsulfonate [Non-ionic photoacid generator (A-44)]

The title compound [Non-ionic photoacid generator (A-44)] was obtained in the same manner as that in Example 4 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, $\delta$(ppm)8.6-8.4(m, 5H), 7.9-7.7(m, 3H), 7.3(d, 1H), 7.1(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Example 45

Synthesis of 4-pentafluorophenoxy-1,8-naphthalimide-(+)-10-camphorsulfonate [Non-ionic photoacid generator (A-45)]

The title compound [Non-ionic photoacid generator (A-45)] was obtained in the same manner as that in Example 5 except that the trifluoromethanesulfonic anhydride (7.4 g, 26 mmol) was changed to (+)-10-camphorsulfonyl chloride (6.5 g, 26 mmol). The product was identified by $^1$H-NMR and $^{19}$ F-NMR. {$^1$H-NMR: 300MHz, Deuterated chloroform, $\delta$(ppm)8.6-8.4(m, 3H), 7.8(d, 1H), 7.1(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, $\delta$(ppm)-146(d, 2F), -149 (t, 1F), -155 (t, 2F)}

Example 46

Synthesis of 4-(3-trifluoromethylphenoxy)-1,8-naphthalimide-(+)-10-camphorsulfonate [Non-ionic photoacid generator (A-46)]

The title compound [Non-ionic photoacid generator (A-46)] was obtained in the same manner as that in Example 6 except that the trifluoromethanesulfonic anhydride (7.4 g, 26 mmol) was changed to (+)-10-camphorsulfonyl chloride

(6.5 g, 26 mmol). The product was identified by $^1$H-NMR and $^{19}$ F-NMR. {$^1$H-NMR: 300MHz, Deuterated chloroform, δ(ppm)8.7-8.5(m, 3H), 7.9-7.6(m, 4H), 7.1-7.0(m, 2H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, δ(ppm)-55(s, 1F), -66(s, 3F), -125(m, 2F), - 133 (m, 1F), -151 (m, 2F)}

Example 47

Synthesis of 4-phenylthio-1,8-naphthalimide-(+)-10-camphorsulfonate [Non-ionic photoacid generator (A-47)]

The title compound [Non-ionic photoacid generator (A-47)] was obtained in the same manner as that in Example 7 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)8.7-8.5(m, 3H), 7.9-7.6(m, 4H), 7.4-7.0(m, 5H), 3.0(d, 1H), 2.5(m, 2H), 2.3(m, 1H), 1.9-1.7(m, 3H), 1.4-1.2(m, 2H), 1.0(s, 3H), 0.8 (s, 3H)}

Example 48

Synthesis of 3-methoxy-1,8-naphthalimide-(+)-10-camphorsulfonate [Non-ionic photoacid generator (A-48)]

The title compound [Non-ionic photoacid generator (A-48)] was obtained in the same manner as that in Example 8 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)8.4-8.3(dd, 2H), 8.0(dd, 2H), 7.8(t, 1H), 4.0(s, 3H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Example 49

Synthesis of 3-(2-ethylhexanoyloxy)-1,8-naphthalimide-(+)-10-camphorsulfonate [Non-ionic photoacid generator (A-49)]

The title compound [Non-ionic photoacid generator (A-49)] was obtained in the same manner as that in Example 9 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)8.6(d, 1H), 8.3(m, 2H), 8.1(s, 1H), 7.8(t, 1H), 3.0(d, 1H), 2.6(m, 1H), 2.5(m, 2H), 2.3(m, 1H), 1.9-1.6(m, 7H), 1.5-1.2(m, 6H), 1.1(t, 3H), 1.0(s, 3H), 0.9(t, 3H), 0.8(s, 3H)}

Example 50

Synthesis of 3-butyroyloxy-1,8-naphthalimide-(+)-10-camphorsulfonate [Non-ionic photoacid generator (A-50)]

The title compound [Non-ionic photoacid generator (A-50)] was obtained in the same manner as that in Example 10 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)8.6(d, 1H), 8.3(m, 2H), 8.1(s, 1H), 7.8(t, 1H), 3.0(d, 1H), 2.6(m, 2H), 2.5(m, 2H), 2.3(m, 1H), 1.9-1.6(m, 7H), 1.4-1.2(m, 2H), 1.0(s, 3H), 0.9(t, 3H), 0.8(s, 3H)}

Example 51

Synthesis of 3-benzoyloxy-1,8-naphthalimide-(+)-10-camphorsulfonate [Non-ionic photoacid generator (A-51)]

The title compound [Non-ionic photoacid generator (A-51)] was obtained in the same manner as that in Example 11 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)8.6(d, 1H), 8.3(m, 2H), 8.1(s, 1H), 8.0-8.9(m, 2H), 7.8(t, 1H), 7.6-7.3(m, 3H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Example 52

Synthesis of 3-pentafluorobenzoyloxy-1,8-naphthalimide-(+)-10-camphorsulfonate [Non-ionic photoacid generator (A-52)]

The title compound [Non-ionic photoacid generator (A-52)] was obtained in the same manner as that in Example 12 except that the trifluoromethanesulfonic anhydride (7.4 g, 26 mmol) was changed to (+)-10-camphorsulfonyl chloride (6.5 g, 26 mmol). The product was identified by $^1$H-NMR and $^{19}$F-NMR. {$^1$H-NMR: 300MHz, Deuterated chloroform, δ(ppm)8.6(d, 1H), 8 . 3 (m, 2H), 8 . 1 (s, 1H), 7.8(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, δ(ppm), -125(m, 2F), -133(m, 1F), -151(m, 2F)}

Example 53

Synthesis of 3,6-di-tert-butyl-1,8-naphthalimide-(+)-10-camphorsulfonate [Non-ionic photoacid generator (A-53)]

The title compound [Non-ionic photoacid generator (A-53)] was obtained in the same manner as that in Example 13 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)7.7(d, 2H), 7.4(d, 2H), 3.0(d, 1H), 2.5(m, 2H), 2.3(m, 1H), 1.9-1.7(m, 3H), 1.4-1.2(m, 2H), 1.0(s, 9H), 1.0(s, 3H), 0.8(s, 3H)}

Example 54

Synthesis of 3,6-dineopentyl-1,8-naphthalimide-(+)-10-camphorsulfonate [Non-ionic photoacid generator (A-54)]

The title compound [Non-ionic photoacid generator (A-54)] was obtained in the same manner as that in Example 14 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)7.7(d, 2H), 7.4(d, 2H), 1.2(q, 2H), 1.0(s, 6H), 0.9(t, 3H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Example 55

Synthesis of 3,6-diadamantyl-1,8-naphthalimide-(+)-10-camphorsulfonate [Non-ionic photoacid generator (A-55)]

The title compound [Non-ionic photoacid generator (A-55)] was obtained in the same manner as that in Example 15 except that the trifluoromethanesulfonic anhydride (7.4 g, 26 mmol) was changed to (+)-10-camphorsulfonyl chloride (6.5 g, 26 mmol). The product was identified by $^1$H-NMR. {$^1$H-NMR: 300MHz, Deuterated chloroform, δ(ppm)7.7(d, 2H), 7.4(d, 2H), 3.0(d, 1H), 2.5(m, 2H), 2.3(m, 1H), 1.9-1.7(m, 3H), 1.4-1.2(m, 2H), 1.2-0.8(m, 21H)}

Example 56

Synthesis of 4-(nonafluorobutyl)-1,8-naphthalimide-(+)-10-camphorsulfonate [Non-ionic photoacid generator (A-56)]

The title compound [Non-ionic photoacid generator (A-56)] was obtained in the same manner as that in Example 16 except that the trifluoromethanesulfonic anhydride (7.4 g, 26 mmol) was changed to (+)-10-camphorsulfonyl chloride (6.5 g, 26 mmol). The product was identified by $^1$H-NMR and $^{19}$F-NMR. {$^1$H-NMR: 300MHz, Deuterated chloroform, δ(ppm)8.8(m, 2H), 8.7(d, 1H), 8.2(d, 1H), 7.9(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, δ(ppm)-76(s, 3F), -100(m, 2F), -116(m, 2F), - 121 (m, 2F)}

Example 57

Synthesis of 4-(heptadecafluoro-n-octyl)-1,8-naphthalimide-(+)-10-camphorsulfonate [Non-ionic photoacid generator (A-57)]

The title compound [Non-ionic photoacid generator (A-57)] was obtained in the same manner as that in Example 17 except that the trifluoromethanesulfonic anhydride (7.4 g, 26 mmol) was changed to (+)-10-camphorsulfonyl chloride (6.5 g, 26 mmol). The product was identified by $^1$H-NMR and $^{19}$F-NMR. {$^1$H-NMR: 300MHz, Deuterated chloroform,

δ(ppm)8.8(m, 2H), 8.7(d, 1H), 8.2(d, 1H), 7.9(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H), $^{19}$F-NMR:300MHz, Deuterated chloroform, δ(ppm)-80(m, 3F), -100(m, 2F), -116(m, 2F), - 118 to -120(m, 8F), -124 (m, 2F)}

Example 58

Synthesis of 4-trimethylsilyl-1,8-naphthalimide-(+)-10-camphorsulfonate [Non-ionic photoacid generator (A-58)]

The title compound [Non-ionic photoacid generator (A-58)] was obtained in the same manner as that in Example 18 except that the trifluoromethanesulfonic anhydride (7.4 g, 26 mmol) was changed to (+)-10-camphorsulfonyl chloride (6.5 g, 26 mmol). The product was identified by $^{1}$H-NMR. {$^{1}$H-NMR: 300MHz, Deuterated chloroform, δ(ppm)8.5-8.4(m, 3H), 7.9(d, 1H), 7.7(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H), 0.4(s, 9H)}

Example 59

Synthesis of 4-chloro-1,8-naphthalimide-(+)-10-camphorsulfonate [Non-ionic photoacid generator (A-59)]

The title compound [Non-ionic photoacid generator (A-59)] was obtained in the same manner as that in Example 19 except that the trifluoromethanesulfonic anhydride (7.4 g, 26 mmol) was changed to (+)-10-camphorsulfonyl chloride (6.5 g, 26 mmol). The product was identified by $^{1}$H-NMR. {$^{1}$H-NMR: 300MHz, Deuterated chloroform, δ(ppm)8.5-8.4(m, 3H), 7.9(d, 1H), 7.7(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Example 60

Synthesis of 4-bromo-1,8-naphthalimide-(+)-10-camphorsulfonate [Non-ionic photoacid generator (A-60)]

The title compound [Non-ionic photoacid generator (A-60)] was obtained in the same manner as that in Example 20 except that the trifluoromethanesulfonic anhydride (7.4 g, 26 mmol) was changed to (+)-10-camphorsulfonyl chloride (6.5 g, 26 mmol). The product was identified by $^{1}$H-NMR. {$^{1}$H-NMR: 300MHz, Deuterated chloroform, δ(ppm)8.5-8.4(m, 3H), 7.9(d, 1H), 7.7(t, 1H), 3.0(d, 1H), 2.5(m, 2H), 2.3(m, 1H), 1.9-1.7(m, 3H), 1.4-1.2(m, 2H), 1.0(s, 3H), 0.8(s, 3H)}

Comparative Example 5

Synthesis of 1,8-naphthalimide-(+)-10-camphorsulfonate

[Non-ionic photoacid generator (A'-5)]

The title compound [Non-ionic photoacid generator (A'-5)] was obtained in the same manner as that in Comparative Example 1 except that the trifluoromethanesulfonic anhydride (7.4 g, 26 mmol) was changed to (+)-10-camphorsulfonyl chloride (6.5 g, 26 mmol).

Comparative Example 6

<Synthesis of Ionic photoacid generator (A'-6)>

An ionic photoacid generator (A'-6) was obtained in the same manner as that in Comparative Example 2 except that the aqueous potassium trifluoromethanesulfonate solution was changed to an aqueous potassium (+)-10-camphorsulfonate solution.

[Chemical 5]

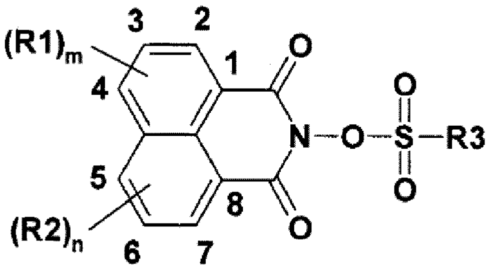

[Table 1]

| | | R1 | R2 | R3 |
|---|---|---|---|---|
| Example 1 | A-1 | 4-isopropoxy | H | -CF3 |
| Example 2 | A-2 | 4-n-propoxy | H | |
| Example 3 | A-3 | 4-(2,2,3,3-tetrafluoro-1-propoxy) | H | |
| Example 4 | A-4 | 4-phenylacetyl | H | |
| Example 5 | A-5 | 4-pentafluorophenoxy | H | |
| Example 6 | A-6 | 4-(3-trifluoromethylphenoxy) | H | |
| Example 7 | A-7 | 4-phenylthio | H | |
| Example 8 | A-8 | 3-methoxy | H | |
| Example 9 | A-9 | 3-(2-ethylhexanoyloxy) | H | |
| Example 10 | A-10 | 3-butyroyloxy | H | |
| Example 11 | A-11 | 3-benzoyloxy | H | |
| Example 12 | A-12 | 3-pentafluorobenzoyloxy | H | |
| Example 13 | A-13 | 3-tert-butyl | 6-tert-butyl | |
| Example 14 | A-14 | 3-neopentyl | 6-neopentyl | |
| Example 15 | A-15 | 3-adamantyl | 6-adamantyl | |
| Example 16 | A-16 | 4-nonafluoro-n-butyl | H | |
| Example 17 | A-17 | 4-(heptadecafluoro-n-octyl) | H | |
| Example 18 | A-18 | 4-trimethylsilyl | H | |
| Example 19 | A-19 | 4-chloro | H | |
| Example 20 | A-20 | 4-bromo | H | |
| Comparative Example 1 | A'-1 | H | H | |

[Table 2]

| | Acid generator | R1 | R2 | R3 |
|---|---|---|---|---|
| Example 21 | A-21 | 4-isopropoxy | H | |
| Example 22 | A-22 | 4-n-propoxy | H | |
| Example 23 | A-23 | 4-(2,2,3,3-tetrafluoro-1-propoxy) | H | |

(continued)

| | Acid generator | R1 | R2 | R3 |
|---|---|---|---|---|
| Example 24 | A-24 | 4-phenylacetyl | H | 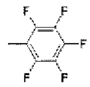 |
| Example 25 | A-25 | 4-pentafluorophenoxy | H | |
| Example 26 | A-26 | 4-(3-trifluoromethylphenoxy) | H | |
| Example 27 | A-27 | 4-phenylthio | H | |
| Example 28 | A-28 | 3-methoxy | H | |
| Example 29 | A-29 | 3-(2-ethylhexanoyloxy) | H | |
| Example 30 | A-30 | 3-butyroyloxy | H | |
| Example 31 | A-31 | 3-benzoyloxy | H | |
| Example 32 | A-32 | 3-pentafluorobenzoyloxy | H | |
| Example 33 | A-33 | 3-tert-butyl | 6-tert-butyl | |
| Example 34 | A-34 | 3-neopentyl | 6-neopentyl | |
| Example 35 | A-35 | 3-adamantyl | 6-adamantyl | |
| Example 36 | A-36 | 4-nonafluoro-n-butyl | H | |
| Example 37 | A-37 | 4-(heptadecafluoro-n-octyl) | H | |
| Example 38 | A-38 | 4-trimethylsilyl | H | |
| Example 39 | A-39 | 4-chloro | H | |
| Example 40 | A-40 | 4-bromo | H | |
| Comparative Example 3 | A'-3 | H | H | |

[Table 3]

| | | R1 | R2 | R3 |
|---|---|---|---|---|
| Example 41 | A-41 | 4-isopropoxy | H | 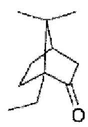 |
| Example 42 | A-42 | 4-n-propoxy | H | |
| Example 43 | A-43 | 4-(2,2,3,3-tetrafluoro-1-propoxy) | H | |
| Example 44 | A-44 | 4-phenylacetyl | H | |
| Example 45 | A-45 | 4-pentafluorophenoxy | H | |
| Example 46 | A-46 | 4-(3-trifluoromethylphenoxy) | H | |
| Example 47 | A-47 | 4-phenylthio | H | |
| Example 48 | A-48 | 3-methoxy | H | |
| Example 49 | A-49 | 3-(2-ethylhexanoyloxy) | H | |
| Example 50 | A-50 | 3-butyroyloxy | H | |
| Example 51 | A-51 | 3-benzoyloxy | H | |
| Example 52 | A-52 | 3-pentafluorobenzoyloxy | H | |
| Example 53 | A-53 | 3-tert-butyl | 6-tert-butyl | |
| Example 54 | A-54 | 3-neopentyl | 6-neopentyl | |
| Example 55 | A-55 | 3-adamantyl | 6-adamantyl | |
| Example 56 | A-56 | 4-nonafluoro-n-butyl | H | |

(continued)

| | | R1 | R2 | R3 |
|---|---|---|---|---|
| Example 57 | A-57 | 4-(heptadecafluoro-n-octyl) | H | |
| Example 58 | A-58 | 4-trimethylsilyl | H | |
| Example 59 | A-59 | 4-chloro | H | |
| Example 60 | A-60 | 4-bromo | H | |
| Comparative Example 5 | A'-5 | H | H | |

<Performance evaluation>

With regard to the performance evaluation of the photoacid generator, obtained non-ionic photoacid generators (A-1) to (A-60), non-ionic photoacid generators (A'-1), (A'-3) and (A'-5), and ionic acid generators (A'-2), (A'-4) and (A'-6) were evaluated for the molar extinction coefficient, the resist curing properties, the thermal decomposition temperature and the solvent solubility in the following manner.

<Molar extinction coefficient>

The synthesized photoacid generator was diluted to 0.25 mmol/L with acetonitrile, and using an ultraviolet-visible spectrophotometer (UV-2550 available from SHIMADZU CORPORATION), the absorbance in a range of 200 nm to 500 nm was measured with a cell having an optical path length (a cell length) of 1 cm. The molar extinction coefficient of i-line (365 nm) ($\varepsilon_{365}$) was calculated from the following equation.

$$\varepsilon_{365}(\mathrm{L \cdot mol^{-1} \cdot cm^{-1}}) = A_{365}/(0.00025\ \mathrm{mol/L} \times 1\ \mathrm{cm})$$

[In the equation, $A_{365}$ represents the absorbance at 365 nm.]

<Resist curing properties>

A resin solution of 75 parts of a phenol resin ("PHENOLITE TD 431" available from DIC Corporation), 25 parts of a melamine curing agent ("CYMEL 300" available from MITSUI CYANAMID Co., Ltd.), 1 part of a synthesized photoacid generator and 200 parts of propylene glycol monomethyl ether acetate (hereinafter, abbreviated as PGMEA) was applied on a glass substrate of 10 cm square under a condition of 10 seconds at 1000 rpm using a spin coater. Then, the applied film was dried under vacuum for 5 minutes at 25°C, after which the film was dried for 3 minutes on an 80°C hot plate to form a resist film with a film thickness of about 3 μm. The whole face of this resist film was exposed with a prescribed dose of ultraviolet light having a wavelength limited by the L-34 filter (a filter cutting light with a wavelength less than 340 nm, available from Kenko Optical Co., Ltd.) using an ultraviolet irradiation device (HMW-661F-01 available from ORC MANUFACTURING CO., LTD.). In this connection, a wavelength of 365 nm was measured for the integrated exposure quantity. Then, the film was subjected to post exposure baking (PEB) for 10 minutes with a 120°C fair wind dryer, after which the film was immersed for 30 seconds in a 0.5% potassium hydroxide solution to be subjected to development, washed immediately with water and dried.

The film thickness of this resist film was measured using a shape measuring microscope (super-depth shape measuring microscope VK-8550, available from KEYENCE

CORPORATION).

In this context, the resist curing properties were evaluated according to the following criteria on the basis of the lowest exposure quantity with which a change in film thickness of the resist film before and after development of within 10% was attained.

○: The lowest exposure quantity is 250 $mJ/cm^2$ or less.
Δ: The lowest exposure quantity is greater than 250 $mJ/cm^2$ and 500 $mJ/cm^2$ or less.
×: The lowest exposure quantity is greater than 500 $mJ/cm^2$.

<Thermal decomposition temperature>

The synthesized photoacid generator was measured for the change in weight under a temperature increasing condition of 10°C/minute from 30°C to 500°C under a nitrogen atmosphere using a thermal gravimetric/differential thermal analyzer (TG/DTA 6200, available from Seiko Instruments Inc.), and a point where the weight was reduced by 2% was defined as the thermal decomposition temperature.

<Solvent solubility>

In a test tube, 0.1 g of the synthesized photoacid generator was placed, and an organic solvent (butyl acetate, toluene or PGMEA) was added thereto in 0.2g portions under a condition where the temperature was adjusted to 25°C until the photoacid generator was completely dissolved. In this connection, in the case where the photoacid generator was not completely dissolved even when 20 g of the solvent was added, the photoacid generator was evaluated as one that was not dissolved.

The non-ionic photoacid generators (A-1) to (A-60) according to the present invention prepared in Examples 1 to 60 and the photoacid generators (A'-1) to (A'-6) for comparison prepared in Comparative Examples 1 to 6 were measured for the molar extinction coefficient, the thermal decomposition temperature and the solvent solubility in the foregoing manner. The results are shown in Tables 4 to 6.

[Table 4]

| | | Molar extinction coefficient ($L \cdot mol^{-1} \cdot cm^{-1}$) | Resist curing properties | Lowest exposure quantity ($mJ/cm^2$) | Thermal decomposition temperature (°C) | Solvent solubility (wt%) | | |
|---|---|---|---|---|---|---|---|---|
| | | | | | | Butyl acetate | Toluene | PGMEA |
| Example 1 | A-1 | 16300 | ○ | 75 | 234 | 8.5 | 9 | 6.5 |
| Example 2 | A-2 | 14000 | ○ | 80 | 238 | 4.5 | 7.5 | 4 |
| Example 3 | A-3 | 12000 | ○ | 60 | 230 | 6 | 12 | 7.5 |
| Example 4 | A-4 | 3000 | ○ | 75 | 240 | 4.5 | 7 | 4 |
| Example 5 | A-5 | 12000 | ○ | 50 | 245 | 17 | 16 | 14 |
| Example 6 | A-6 | 16000 | ○ | 55 | 239 | 14 | 13 | 11 |
| Example 7 | A-7 | 20000 | ○ | 75 | 240 | 7 | 7 | 5 |
| Example 8 | A-8 | 7500 | ○ | 75 | 243 | 8 | 7.5 | 6 |
| Example 9 | A-9 | 1500 | ○ | 40 | 231 | >30 | >30 | >30 |
| Example 10 | A-10 | 1600 | ○ | 55 | 234 | >30 | >30 | >30 |
| Example 11 | A-11 | 1400 | ○ | 60 | 241 | 25 | 23 | 20 |
| Example 12 | A-12 | 1300 | ○ | 50 | 242 | >30 | >30 | >30 |
| Example 13 | A-13 | 6500 | ○ | 45 | 245 | 8 | 7.5 | 8 |
| Example 14 | A-14 | 6200 | ○ | 50 | 248 | 18 | 10 | 14 |
| Example 15 | A-15 | 6000 | ○ | 55 | 250 | 7.5 | 8.5 | 7 |
| Example 16 | A-16 | 40 | Δ | 280 | 239 | 25 | 28 | 22 |
| Example 17 | A-17 | 80 | Δ | 260 | 242 | >30 | >30 | >30 |
| Example 18 | A-18 | 5500 | ○ | 60 | 240 | 7.5 | 9 | 8 |
| Example 19 | A-19 | 6000 | ○ | 85 | 220 | 5 | 10 | 6 |
| Example 20 | A-20 | 8500 | ○ | 90 | 230 | 4.5 | 8 | 4 |
| Comparative Example 1 | A'-1 | 330 | Δ | 310 | 225 | 2 | 4 | 2.4 |
| Comparative Example 2 | A'-2 | 80 | × | 880 | 340 | <0.5 | <0.5 | >30 |

[Table 5]

| | | Molar extinction coefficient ($L \cdot mol^{-1} \cdot cm^{-1}$) | Resist curing properties | Lowest exposure quantity ($mJ/cm^2$) | Thermal decomposition temperature (°C) | Solvent solubility (wt%) | | |
|---|---|---|---|---|---|---|---|---|
| | | | | | | Butyl acetate | Toluene | PGMEA |
| Example 21 | A-21 | 16300 | ○ | 140 | 236 | 4 | 3.5 | 4.5 |
| Example 22 | A-22 | 14000 | ○ | 140 | 240 | 3.5 | 6 | 3 |
| Example 23 | A-23 | 12000 | ○ | 120 | 232 | 7.5 | 10 | 7 |
| Example 24 | A-24 | 3000 | ○ | 135 | 242 | 3.5 | 5.5 | 3.2 |
| Example 25 | A-25 | 12000 | ○ | 90 | 247 | 14 | 13 | 11 |
| Example 26 | A-26 | 16000 | ○ | 100 | 241 | 11 | 10 | 9 |
| Example 27 | A-27 | 20000 | ○ | 140 | 242 | 5.5 | 5.5 | 4 |
| Example 28 | A-28 | 7500 | ○ | 140 | 240 | 6.5 | 6 | 5 |
| Example 29 | A-29 | 1500 | ○ | 80 | 230 | 25 | 23 | 18 |
| Example 30 | A-30 | 1600 | ○ | 100 | 235 | 18 | 20 | 14 |
| Example 31 | A-31 | 1400 | ○ | 120 | 243 | 20 | 18 | 16 |
| Example 32 | A-32 | 1300 | ○ | 90 | 244 | 24 | 22 | 19 |
| Example 33 | A-33 | 6500 | ○ | 85 | 245 | 6.5 | 6 | 6 |
| Example 34 | A-34 | 6200 | ○ | 90 | 250 | 14 | 8 | 11 |
| Example 35 | A-35 | 6000 | ○ | 100 | 251 | 6 | 9 | 5.5 |
| Example 36 | A-36 | 40 | Δ | 350 | 239 | 14 | 11 | 13 |
| Example 37 | A-37 | 80 | Δ | 310 | 244 | 25 | 28 | 23 |
| Example 38 | A-38 | 5500 | ○ | 110 | 241 | 5.5 | 6 | 5.5 |
| Example 39 | A-39 | 6000 | ○ | 160 | 225 | 3 | 5 | 3.5 |
| Example 40 | A-40 | 8500 | ○ | 180 | 243 | 3 | 4.5 | 3.2 |
| Comparative Example 3 | A'-3 | 330 | Δ | 360 | 228 | 1.6 | 3.2 | 1.9 |
| Comparative Example 4 | A'-4 | 80 | × | 950 | 330 | <0.5 | <0.5 | >30 |

[Table 6]

| | | Molar extinction coefficient ($L \cdot mol^{-1} \cdot cm^{-1}$) | Resist curing properties | Lowest exposure quantity ($mJ/cm^2$) | Thermal decomposition temperature (°C) | Solvent solubility (wt%) | | |
|---|---|---|---|---|---|---|---|---|
| | | | | | | Butyl acetate | Toluene | PGMEA |
| Example 41 | A-41 | 16300 | ○ | 210 | 239 | 1.7 | 2 | 1.8 |
| Example 42 | A-42 | 14000 | ○ | 220 | 245 | 1.5 | 2 | 1.2 |
| Example 43 | A-43 | 12000 | ○ | 180 | 231 | 3 | 3.5 | 3 |
| Example 44 | A-44 | 3000 | ○ | 210 | 233 | 1.4 | 1.8 | 1.8 |
| Example 45 | A-45 | 12000 | ○ | 140 | 245 | 4 | 4 | 3.5 |
| Example 46 | A-46 | 16000 | ○ | 160 | 243 | 3.5 | 3.3 | 2.8 |
| Example 47 | A-47 | 20000 | ○ | 210 | 244 | 1.8 | 1.7 | 1.3 |
| Example 48 | A-48 | 7500 | ○ | 210 | 242 | 2 | 1.9 | 1.5 |
| Example 49 | A-49 | 1500 | ○ | 120 | 232 | 11 | 9 | 8 |
| Example 50 | A-50 | 1600 | ○ | 150 | 237 | 9 | 7 | 7.2 |
| Example 51 | A-51 | 1400 | ○ | 180 | 245 | 6 | 6 | 5 |
| Example 52 | A-52 | 1300 | ○ | 140 | 245 | 12 | 10 | 7.5 |
| Example 53 | A-53 | 6500 | ○ | 130 | 247 | 2 | 1.9 | 1.7 |
| Example 54 | A-54 | 6200 | ○ | 140 | 252 | 4.5 | 2.5 | 3.5 |
| Example 55 | A-55 | 6000 | ○ | 160 | 253 | 2 | 2.1 | 1.7 |
| Example 56 | A-56 | 40 | Δ | 440 | 241 | 5 | 5.5 | 5 |
| Example 57 | A-57 | 80 | Δ | 410 | 246 | 9.5 | 10 | 9 |
| Example 58 | A-58 | 5500 | ○ | 170 | 243 | 2.5 | 3 | 2.5 |
| Example 59 | A-59 | 6000 | ○ | 240 | 228 | 1.5 | 2.5 | 1.5 |
| Example 60 | A-60 | 8500 | ○ | 250 | 240 | 1.1 | 2 | 1.5 |
| Comparative Example 5 | A'-5 | 330 | Δ | 480 | 230 | <0.5 | <0.6 | <0.5 |
| Comparative Example 6 | A'-6 | 80 | × | 990 | 330 | <0.5 | <0.5 | 10 |

As apparent from Tables 4 to 6, it has been found that each of the non-ionic photoacid generators (A) in Examples 1 to 15, 18 to 35, 38 to 55 and 58 to 60 of the present invention has a molar extinction coefficient of i-line (365 nm) greater than that of the photoacid generator in Comparative Example, and is satisfactory in resist curing properties. Moreover, in any example, the non-ionic photoacid generator is excellent in solubility to a solvent, and in Examples 3, 5, 6, 8 to 12, 16 to 18, 23, 25, 26, 28 to 32, 36 to 38, 43, 45, 46, 48 to 52 and 56 to 58 where R1 or R2 is a carbonyloxy group, a silyl group or a functional group containing a fluorine atom, the non-ionic photoacid generator is especially excellent therein. In Examples 13 to 15, 33 to 35 and 53 to 55 where R1 and R2 are the same as each other and are the same alkyl group allowing the carbon atom bonded to a naphthalene ring to be a quaternary carbon atom, it has been found that the non-ionic photoacid generator is excellent in balance between curing properties and solubility. Moreover, in any example, it has been found that the thermal decomposition temperature is 220°C or higher and the non-ionic photoacid generator has sufficient stability.

On the other hand, in Comparative Examples 1, 3 and 5 where conventionally known non-ionic photoacid generators are used and in Comparative Examples 2, 4 and 6 where ionic acid generators are used, the resist film has low sensitivity to i-line, and also, it cannot be said that the resist curing properties are sufficient. Moreover, in Comparative Examples 1, 3 and 5, it has been found that the solubility to a solvent is insufficient.

INDUSTRIAL APPLICABILITY

The non-ionic photoacid generator (A) according to the present invention is suitable as a photoacid generator used for a positive type resist, a resist film, a liquid resist, a negative type resist, a resist for MEMS, a photosensitive material, a nanoimprint material, a material for micro-photofabrication, or the like. Moreover, the resin composition (Q) for photolithography according to the present invention is suitably provided for above-described applications.

## Claims

1. A non-ionic photoacid generator (A), being expressed by the following general formula (1):

[Chemical 1]

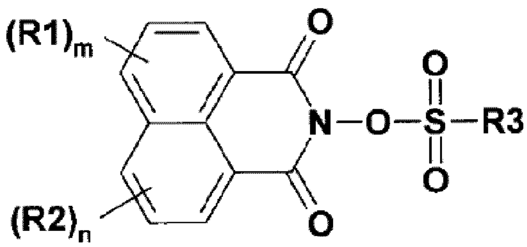

(1)

[In formula (1), R1 and R2 each independently represents an alkyl group having 1 to 18 carbon atoms or a fluoroalkyl group having 1 to 18 carbon atoms, an alkenyl group having 2 to 18 carbon atoms, an alkynyl group having 2 to 18 carbon atoms, an aryl group having 6 to 18 carbon atoms, a silyl group, an alkoxy group or aryloxy group represented by $R^{11}O$-, an alkylthio group or arylthio group represented by $R^{12}S$-, a sulfinyl group represented by $R^{13}SO$-, a sulfonyl group represented by $R^{14}SO_2$-, an alkylcarbonyl group or arylcarbonyl group represented by $R^{15}CO$-, a carbonyloxy group represented by $R^{16}COO$-, an oxycarbonyl group represented by $R^{17}OCO$-, a carbonate group represented by $R^{18}OCOO$-, a urethane group represented by $R^{19}NHCOO$-, or a halogen atom; m and n respectively represent the number of R1s and R2s, each number being an integer from 0 to 3, and the total number (m + n) of R1s and R2s being an integer from 1 to 6. The m number of R1s and the n number of R2s may each be the same or different. R3 represents a hydrocarbon group having 1 to 18 carbon atoms (wherein one part or all of hydrogen atoms may be substituted by fluorine atoms).]

2. The non-ionic photoacid generator (A) according to claim 1, wherein the number of R1 and R2 satisfy the equations of m = 1 and n = 0 or the equations of m = 1 and n = 1, in the general formula (1).

3. The non-ionic photoacid generator (A) according to claim 1 or 2, wherein at least any one of R1 and R2 is an alkyl

group having 1 to 6 carbon atoms, a fluoroalkyl group having 1 to 6 carbon atoms, a silyl group, an alkoxy group or aryloxy group represented by $R^{11}O$-, an alkylthio group or arylthio group represented by $R^{12}S$-, or a carbonyloxy group represented by $R^{16}COO$-, in the general formula (1).

**4.** The non-ionic photoacid generator (A) according to any one of claims 1 to 3, wherein R3 is a linear alkyl group having 1 to 18 carbon atoms, a branched-chain alkyl group having 1 to 18 carbon atoms, a cycloalkyl group having 3 to 18 carbon atoms, $CF_3$, $C_2F_5$, $C_3F_7$, $C_4F_9$, or $C_6F_5$, in the general formula (1).

**5.** A resin composition (Q) for photolithography, comprising a non-ionic photoacid generator (A) according to any one of claims 1 to 4.

## INTERNATIONAL SEARCH REPORT

International application No.
PCT/JP2014/003545

A. CLASSIFICATION OF SUBJECT MATTER
*C09K3/00*(2006.01)i, *C07D221/14*(2006.01)i, *C08K5/42*(2006.01)i, *C08L101/00*(2006.01)i, *G03F7/004*(2006.01)i, *G03F7/038*(2006.01)i, *H01L21/027*(2006.01)i

According to International Patent Classification (IPC) or to both national classification and IPC

B. FIELDS SEARCHED

Minimum documentation searched (classification system followed by classification symbols)
C09K3/00, C07D221/14, C08K5/42, C08L101/00, G03F7/004, G03F7/038, H01L21/027

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2011/087011 A1 (ADEKA Corp.), 21 July 2011 (21.07.2011), claims 1 to 3; paragraphs [0001] to [0002], [0052] to [0053], [0064] to [0070](compounds S-1, S-4, S-10, S-13, S-19, S-21, S-25, S-28, S-39, S-40, S-50, S-51); paragraphs [0113], [0116], [0125] & US 2012/0289697 A1 & EP 2524914 A1 & CN 102712599 A & TW 201139380 A & KR 10-2012-0114353 A | 1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 September, 2014 (17.09.14) | 14 October, 2014 (14.10.14) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No. PCT/JP2014/003545

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2004-217748 A (Konica Minolta Holdings, Inc.), 05 August 2004 (05.08.2004), claim 1; paragraph [0033]; paragraph [0035] (Photoacid generators A-1-9 to A-1-16); paragraph [0184], table 3; paragraph [0195], table 7 (sample no.9 to 12); paragraph [0200], table 8 (sample no.9 to 12) (Family: none) | 1-4<br>5 |
| X<br>A | JP 2004-243676 A (Konica Minolta Holdings, Inc.), 02 September 2004 (02.09.2004), claim 1; paragraph [0128], table 5; paragraph [0129](Initiator 1) (Family: none) | 1-4<br>5 |
| P,X | WO 2014/084269 A1 (ADEKA Corp.), 05 June 2014 (05.06.2014), claims 1 to 4, 6; paragraphs [0001] to [0002], [0036] to [0037](compound no.1, 2, 4, 5); paragraph [0039](compound no.20 to 34); paragraph [0040](compound no.37) (Family: none) | 1-5 |
| P,X | KR 10-2014-1400390 B1 (LG DISPLAY Co., Ltd.), 30 May 2014 (30.05.2014), claims 1 to 2 (chemical formulae 1, 2); paragraphs [0001], [0028] to [0033] (Family: none) | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 50151997 A
- JP 9118663 A
- JP 6067433 A
- JP 10213899 A